# EUROPEAN PATENT SPECIFICATION

(11) **EP 3 337 518 B1**
(45) Date of publication and mention of the grant of the patent: **04.11.2026**
(21) Application number: 16837738.0
(22) Date of filing: 17.08.2016
(51) Int. Cl.: C07K 14/47, C12N 15/85, A61K 48/00, A61P 9/04

(54) **BAG3 COMPOSITIONS FOR USE IN ENHANCING CARDIAC PERFORMANCE IN SUBJECTS SUFFERING FROM HEART FAILURE**
BAG3-ZUSAMMENSETZUNGEN ZUR VERWENDUNG IN DER VERBESSERUNG DER HERZLEISTUNG BEI PERSONEN MIT HERZINSUFFIZIENZ
COMPOSITIONS BAG3 POUR L'UTILISATION DANS L'AMÉLIORATION LES PERFORMANCES CARDIAQUES CHEZ LES SUJETS SOUFFRANT D'INSUFFISANCE CARDIAQUE

(30) Priority: 17.08.2015 US 201562205990 P
(43) Date of publication of application: 27.06.2018
(73) Proprietor: Temple University Of The Commonwealth System Of Higher Education, Philadelphia, PA 19122-6029 (US)
(72) Inventor: FELDMAN, Arthur, M., Wynnewood, PA 19096 (US); CHEUNG, Joseph, Y., Bryn Mawr, PA 19010 (US)
(74) Representative: Patentanwälte Ruff, Wilhelm, Beier, Dauster & Partner mbB
(86) International application number: PCT/US2016/047305
(87) International publication number: WO 2017/031182

(56) References cited:
- WO-A1-2012/107580
- WO-A2-2015/117010
- TIJANA KNEZEVIC ET AL: "BAG3: a new player in the heart failure paradigm", HEART FAILURE REVIEWS, vol. 20, no. 4, 30 April 2015 (2015-04-30), NL, pages 423 - 434, XP055366734, ISSN: 1382-4147, DOI: 10.1007/s10741-015-9487-6

## Description

### CROSS-REFERENCE TO RELATED APPLICATIONS

This application claims the benefit of the filing of U.S. Provisional Application No. 62/205,990, which was filed on August 17, 2015. For the purpose of any U.S. application that may claim the benefit of U.S. Provisional Application No. 62/205,990,

### FIELD OF THE INVENTION

The present invention relates to compositions for enhancing cardiac performance in heart failure. Such compositions, which include comprising nucleic acids encoding Bcl2-associated athanogene 3 (BAG3), can be administered to a subject suffering from heart failure.

### BACKGROUND

Heart failure (HF) affects over 5 million individuals in the U.S. and over 23 million individuals worldwide and is an important cause of both morbidity and mortality. One form of heart failure, heart failure due to reduced ejection fraction (HFrEF), occurs when the heart's ability to contract is impaired. Both nonischemic and ischemic cardiomyopathy can give rise to HFrEF. Currently available treatments include lifestyle changes, drug therapies and mechanical devices. Despite advances in therapy and management, both nonischemic and ischemic cardiomyopathy remain progressive disorders. Patients who are refractile to standard therapies may be candidates for heart transplantation. Heart transplantation is limited both by the scarcity of donor hearts available and the need for the recipient to remain on immunosuppressive treatment. There is a continuing need for new treatments for both nonischemic and ischemic cardiomyopathy due to HFrEF. WO 2015/117010 A2 discloses an agent for treating a patient suffering from, or, at risk of developing a muscle related disease or disorder, wherein the agent modulates expression or amount of BAG3 molecules, proteins or peptides thereof in a target cell or tissue, as compared to normal control.

### SUMMARY

The present invention is defined by the claims. Provided herein are methods and compositions relating to the treatment and prevention of heart failure. The methods can include methods of enhancing cardiac performance in a subject having or at risk for heart failure by administering a composition comprising a nucleic acid encoding a BAG3 polypeptide or fragment thereof. In some embodiments, the patient is suffering from HFrEF. In some embodiments, the patient is suffering from nonischemic cardiomyopathy. In some embodiments, the patient is suffering from ischemic cardiomyopathy. In some embodiments, the methods can include administering a composition comprising a BAG3 -modulating agent. A BAG3-modulating agent can be an agent that specifically increases BAG3 expression or activity in a cardiac myocyte. In some embodiments, a BAG3 modulating agent can be a nucleic acid encoding a BAG3 polypeptide or fragment thereof. In some embodiments, a BAG3 modulating agent can be a nucleic acid encoding a CRISPR-associated endonuclease and a guide RNA that is complementary to a target sequence in a BAG3 polypeptide or fragment thereof.

### BRIEF DESCRIPTION OF THE DRAWINGS

These and other features and advantages of the present invention will be more fully disclosed in, or rendered obvious by, the following detailed description of the preferred embodiment of the invention, which is to be considered together with the accompanying drawings wherein like numbers refer to like parts and further wherein:

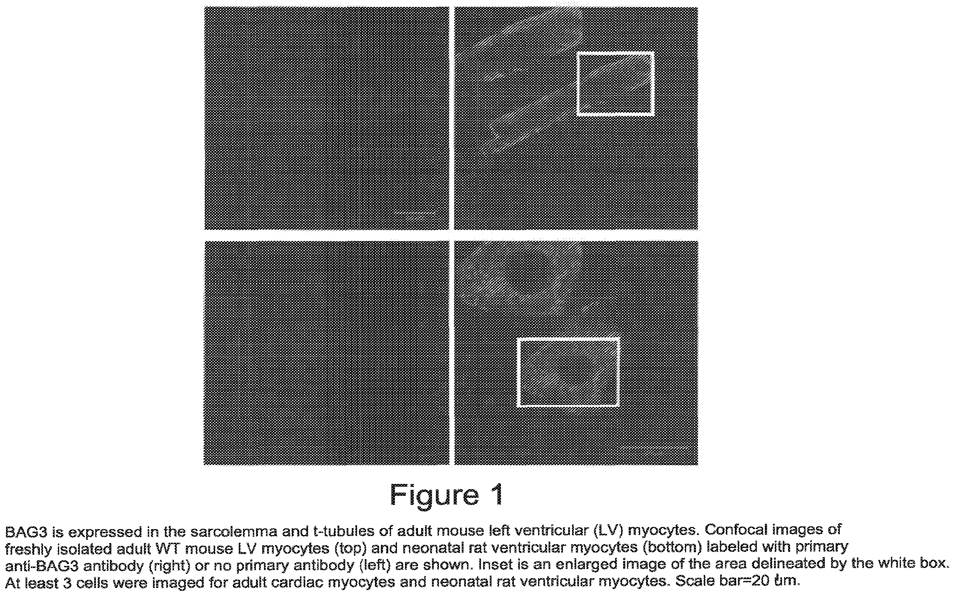

Fig. 1 shows that BAG3 is expressed in the sarcolemma and t-tubules of normal adult mouse left ventricular (LV) myocytes and in the cytoplasm of neonatal myoctes. Confocal images of freshly isolated adult WI mouse LV myocytes labeled with primary anti-BAG3 antibody (1B) or no primary antibody (1A) and neonatal rat ventricular myocytes labeled with primary anti-BAG3 antibody (1D) or no primary antibody (1C) (bottom) are shown. Inset is an enlarged image of the area delineated by the white box. At least 3 cells were imaged for adult cardiac myocytes and neonatal rat ventricular myocytes. Scale bar = 20 µM.

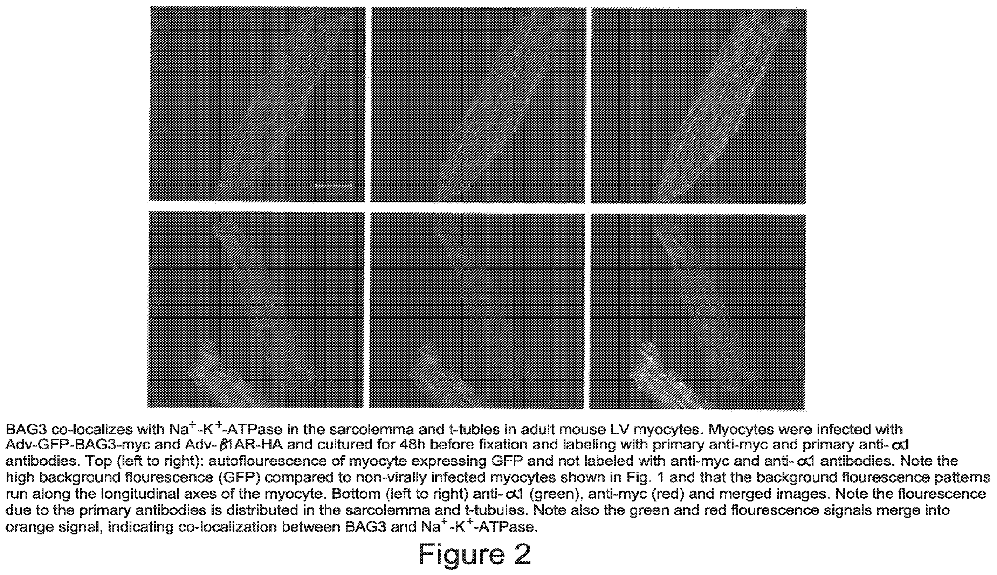

Fig. 2 shows that BAG3 co-localizes with Na+-K+-ATPase in the sarcolemma and t-tubules in adult mouse LV myocytes. Myocytes were infected with Adv-GFP-BAG3-myc and Adv-β1AR-HA and cultured for 48h before fixation and labeling with primary anti-myc and primary anti-α1 antibodies. Top (left to right): autofluorescence of myocytes expressing GFP and not labeled with anti-myc and anti-α1 antibodies (2A). Note the high background fluorescence (GFP) compared to non-virally infected myocytes shown in Fig. 1 and that the background fluorescence patterns run along the longitudinal axes of the myocyte. Fig. 2B shows the same myocyte as in 2A labeled with anti-myc antibody. Fig. 2C shows a merged image of Figs. 2A and 2B. Fig. 2D shows a GFP-expressing myocyte labeled with anti-α1 antibody (green). Fig.2E shows the same GFP-expressing myocyte as in Fig. 2D labeled with anti-myc antibody (red). Fig. 2F shows a merged image of Figs. 2D and 2E. Note the fluorescence due to the primary antibodies is distributed in the sarcolemma and t-tubules. Note also the green and red fluorescence signals merge into orange signal, indicating co-localization between BAG3 and Na+-K+-ATPase.

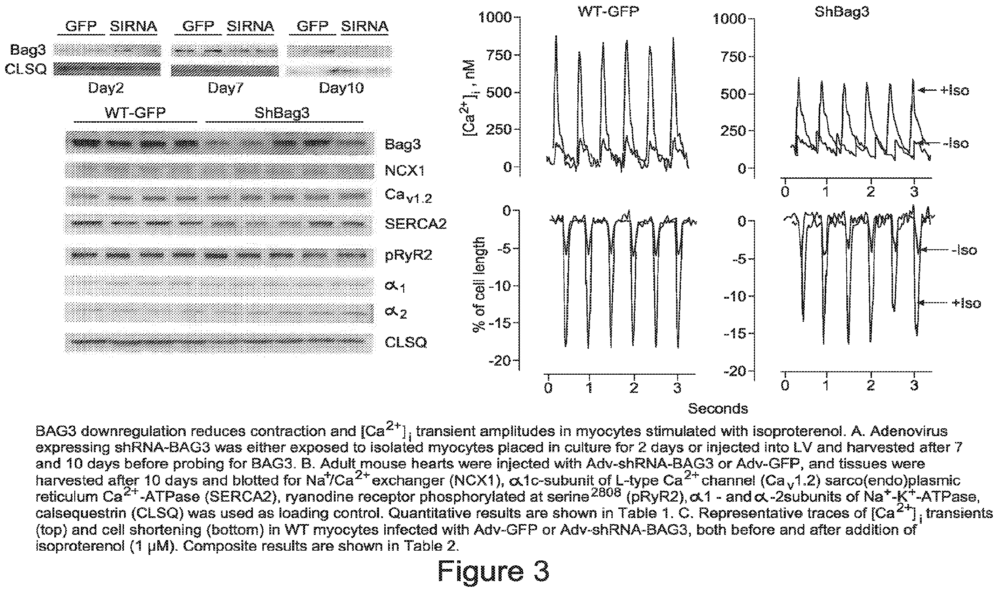

Fig. 3 shows that BAG3 downregulation in adult myocytes reduces contraction and [Ca2+]ᵢ transient amplitudes in myocytes stimulated with isoproterenol. Fig. 3A. Adenovirus expressing shRNA-BAG3 was either exposed to isolated myocytes placed in culture for 2 days or injected into LV and harvested after 7 and 10 days before probing for BAG3. Fig. 3B. Adult mouse hearts were injected with Adv-shRNA-BAG3 or Adv-GFP, and tissues were harvested after 10 days and blotted for Na+/ Ca²⁺ exchanger (NCX1), α1c-subunit of L-type Ca²⁺ channel (Cav1.2), sarco(endo)plasmic reticulum Ca²⁺-ATPase (SERCA2), ryanodine receptor phosphorylated at serine2808 (pRyR2), α1- and α-2subunits of Na+-K+-ATPase, and calsequestrin (CLSQ) was used as loading control. Quantitative results are shown in Table 1. Fig. 3C. Representative traces of [Ca²⁺]ᵢ transients in WT myocytes infected with Adv-GFP, both before and after addition of isoproterenol (1 µM). Fig. 3D. Representative traces of cell shortening in WT myocytes infected with Adv-GFP, both before and after addition of isoproterenol (1 µM). Fig. 3E. Representative traces of [Ca2+]ᵢ transients in myocytes infected with Adv-shRNA-BAG3, both before and after addition of isoproterenol (1 µM). Fig. 3F. Representative traces of cell shortening in myocytes infected with Adv-shRNA-BAG3, both before and after addition of isoproterenol (1 µM). Composite results are shown in Table 2.

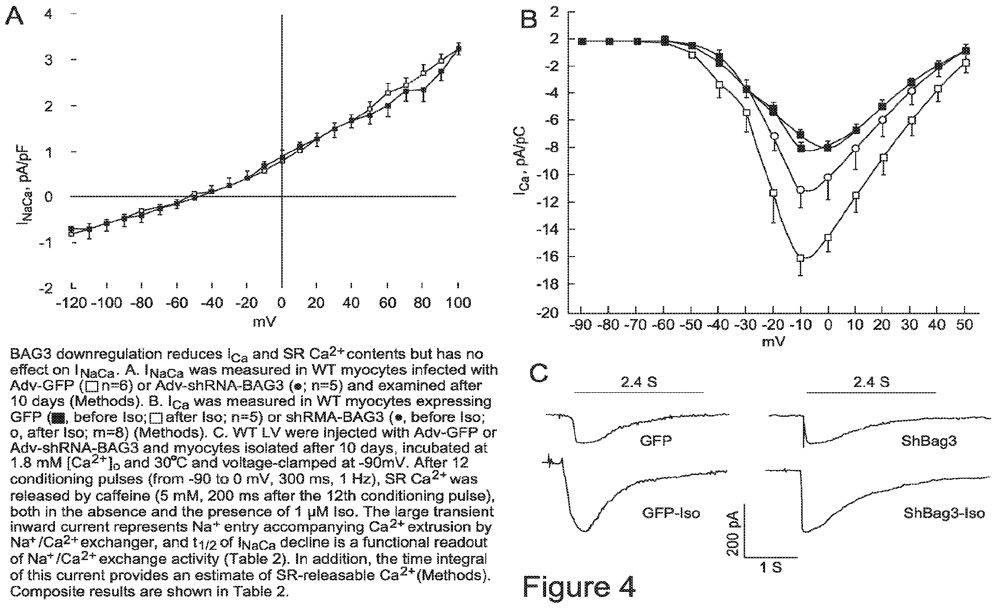

Fig. 4 shows that BAG3 downregulation in adult myocytes reduces I_{c}, and SR Ca²⁺ contents but has no effect on I_{Naca}. Fig. 4A. I_{NaCa} was measured in WT myocytes infected with Adv-GFP (□; n=6) or Adv-shRNA-BAG3 (•; n=5) and examined after 10 days (Methods). Fig. 4B. I_{Ca} was measured in WT myocytes expressing GFP (_{▪}, before Iso; □, after Iso; n=5) or shRNA-BAG3 (-.before Iso; o, after Iso; n=8) (Methods). Fig. 4C. WT LV were injected with Adv-GFP or Adv-shRNA-BAG3 and myocytes isolated after 10 days, incubated at 1.8 mM [Ca2+]_{∘} and 30°C and voltage-clamped at -90 mV. After 12 conditioning pulses (from -90 to 0 mV, 300 ms, 1 Hz), SR Ca²⁺ was released by caffeine (5 mM, 200 ms after the 12^{th} conditioning pulse), both in the absence and presence of 1 µM Iso. The large transient inward current represents Na+ entry accompanying Ca²⁺ extrusion by Na+/ Ca²⁺ exchanger, and t_{1/2} of I_{Naca} decline is a functional readout of Na+/ Ca²⁺ exchange activity (Table 2). In addition, the time integral of this current provides an estimate of SR-releasable Ca²⁺ (Methods). Composite results are shown in Table 2.

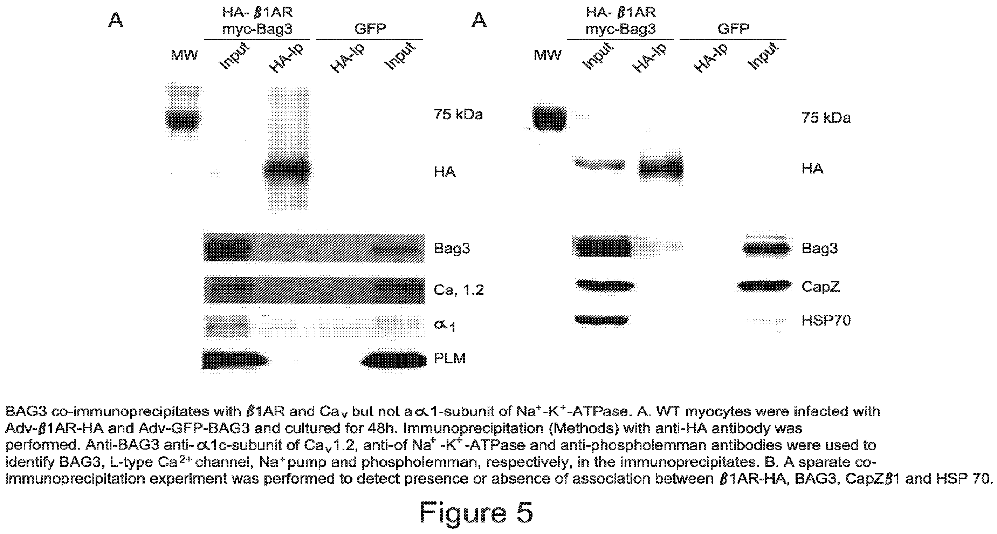

Fig. 5 shows the BAG3 co-immunoprecipitates with β1AR and Cav1.2 but not al-subunit of Na+-K+-ATPase. Fig. 5A. WT myocytes were infected with Adv-β1AR-HA and Adv-GFP-BAG3-myc or Adv-GFP and cultured for 48h. Immunoprecipitation (Methods) with anti-HA antibody was performed. Anti-BAG3, anti-α1c-subunit of Cav1.2, anti-α1-subunit of Na+-K+-ATPase and anti-phospholemman antibodies were used to identify BAG3, L-type Ca²⁺channel, Na+ pump and phospholemman, respectively, in the immunoprecipitates. Fig. 5B. A separate co-immunoprecipitation experiment was performed to detect presence or absence of association between β1AR-HA, BAG3, CapZβ1 and Hsp70.

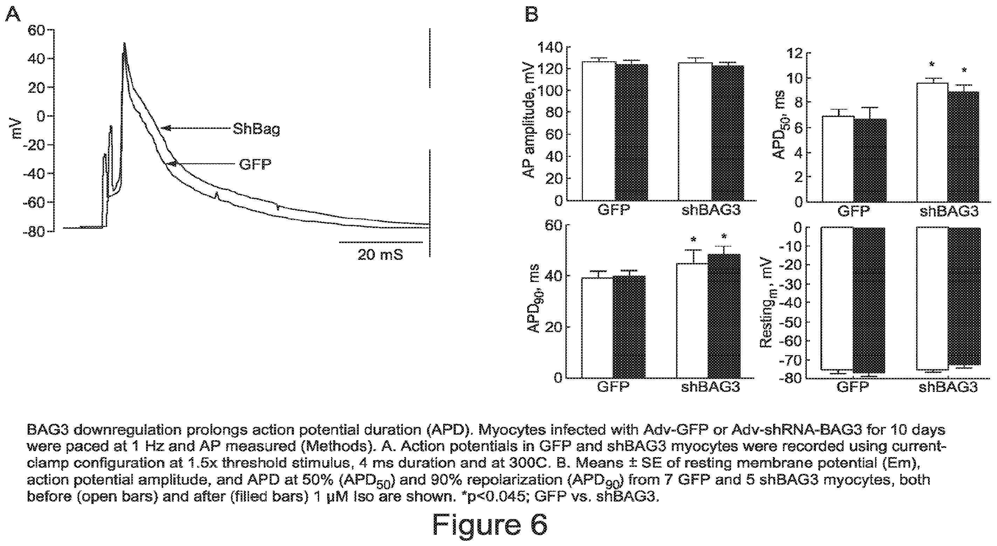

Fig. 6 shows that BAG3 downregulation in adult myocytes prolongs action potential duration (APD). Myocytes infected with Adv-GFP or Adv-shRNA-BAG3 for 10 days were paced at 1 Hz and AP measured (Methods). Fig. 6A. Action potentials in GFP and shBAG3 myocytes were recorded using current-clamp configuration at 1.5x threshold stimulus, 4 ms duration and at 300C. Fig. 6B. Means ± SE action potential amplitude from 7 GFP and 5 shBAG3 myocytes, both before (open bars) and after (filled bars) 1 µM Iso. Fig. 6C. Means ± SE APD at 50% (APD₅₀) from 7 GFP and 5 shBAG3 myocytes, both before (open bars) and after (filled bars) 1 µM Iso. Fig. 6D. Means ± SE APD at 90% repolarization (APD₉₀) from 7 GFP and 5 shBAG3 myocytes, both before (open bars) and after (filled bars) 1 µM Iso. Fig. 6E. Means ± SE of resting membrane potential (Em) from 7 GFP and 5 shBAG3 myocytes, both before (open bars) and after (filled bars) 1 µM Iso. *p<0.045; GFP vs. shB AG3.

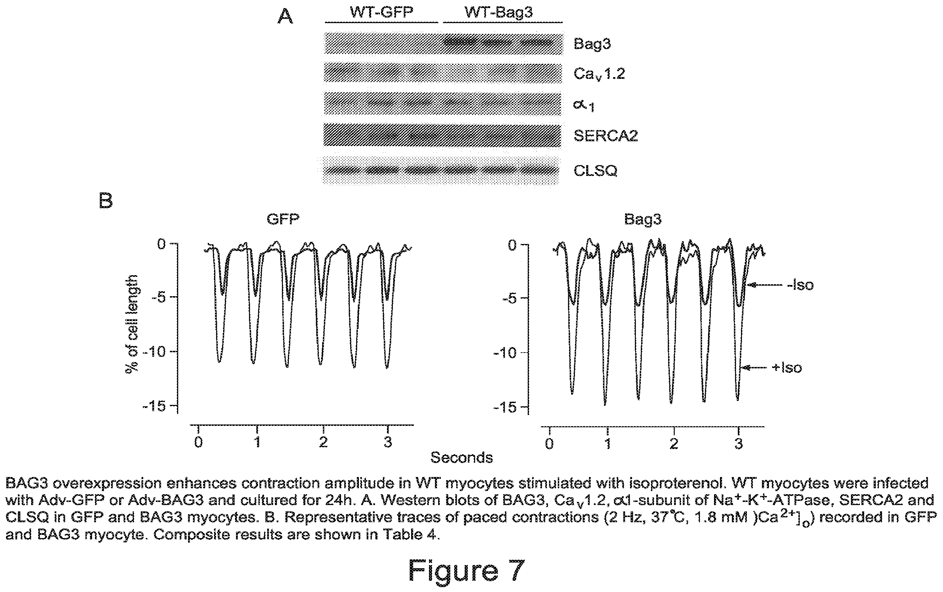

Fig. 7 shows that BAG3 overexpression enhances contraction amplitude in WT adult myocytes stimulated with isoproterenol. WT myocytes were infected withAdv-GFP or Adv- BAG3 and cultured for 24h. Fig. 7A. Western blots of BAG3, Ca, 1.2, a.l-subunit of Na+-K+- ATPase, SERCA2 and CLSQ in GFP and BAG3 myocytes. Fig. 7B. Representative traces of paced contractions (2Hz, 37°C, 1.8 mM [Ca2+]_{∘} recorded in GFP myocyte. Fig. 7C. Representative traces of paced contractions (2Hz, 37°C, 1.8 mM [Ca2+]_{∘} recorded in BAG3 myocyte. Composite results are shown in Table 4.

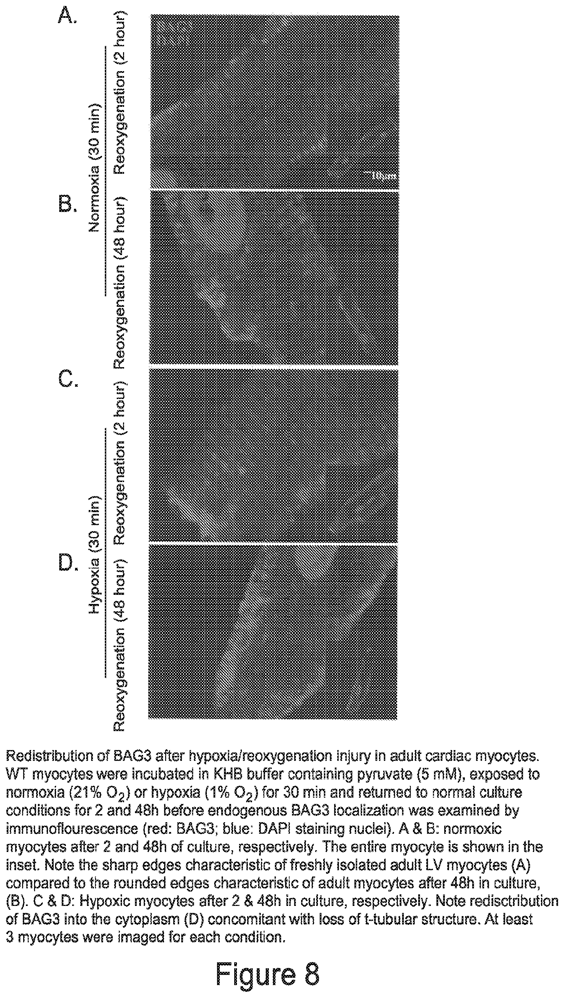

Fig. 8 shows the redistribution of BAG3 after hypoxia/reoxygenation injury in adult cardiac myocytes. WT myocytes were incubated in KHB buffer containing pyruvate (5 mM), exposed to normoxia (21% O₂) or hypoxia (1% O₂) for 30 min and returned to normal culture conditions for 2 and 48h before endogenous BAG3 localization was examined by immunofluorescence (red: BAG3; blue: DAPI staining nuclei). Fig. 8A: Normoxic myocytes after 2 of culture. Fig. 8B: Normoxic myocytes after 48h of culture. The entire myocyte is shown in the inset. Note the sharp edges characteristic of freshly isolated adult LV myocytes (Fig.8A) compared to the rounded edges characteristic of adult myocytes after 48h in culture (Fig.8 C). Hypoxic myocytes after 2 of culture. Fig. 8D: hypoxic myocytes after 48h of culture. Note redistribution of BAG3 into the cytoplasm (Fig. 8D) concomitant with loss of t- tubular structure. At least 3 myocytes were imaged for each condition.

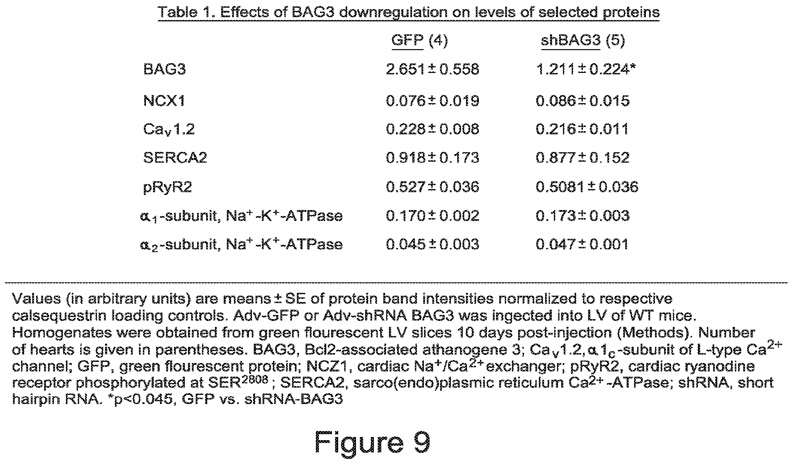

Fig. 9 shows Table 1: Effects of BAG3 regulation on levels of selected proteins.

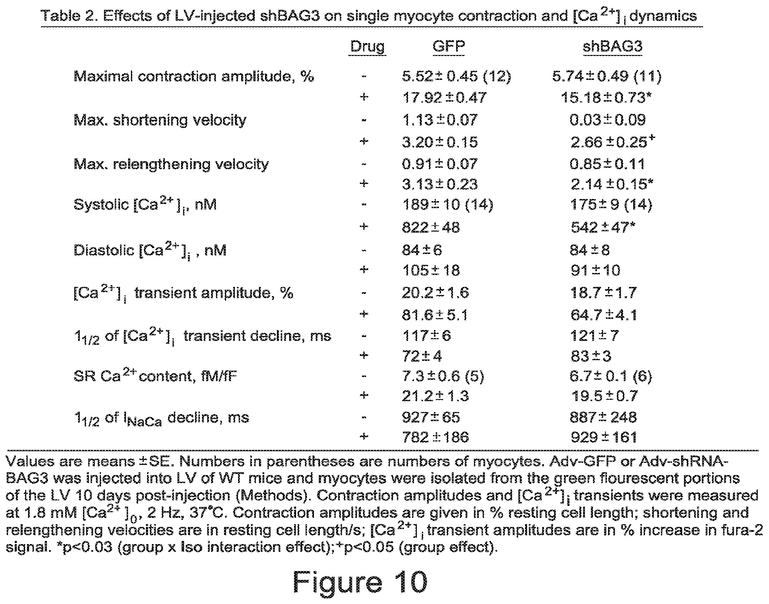

Fig. 10 shows Table 2: Effects of LV- injected shBAG3 on single adult myoocyte contraction and [Ca^{2+]}ᵢ dynamics.

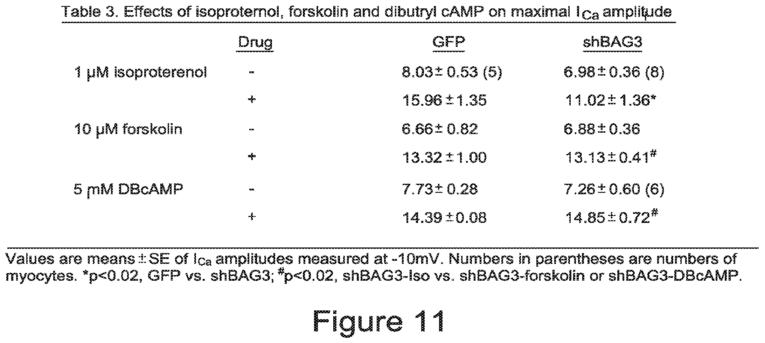

Fig. 11 shows Table 3: Effects of isoproterenol, forskolin and dibutyl cAMP on maximal I_{Ca} amplitude.

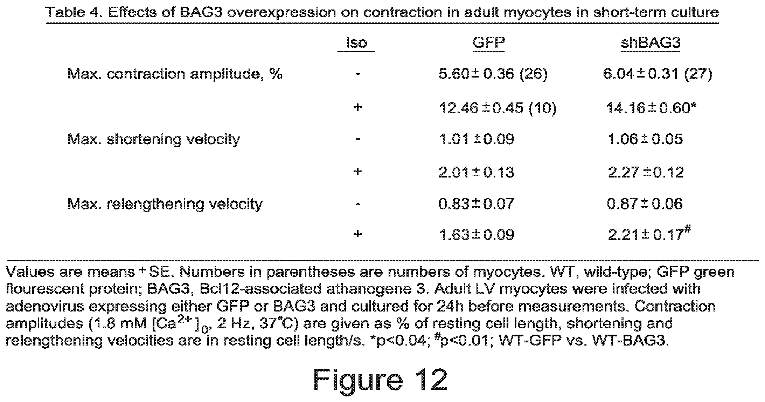

Fig. 12 shows Table 4: Effects of BAG3 overexpression on contraction adult myocytes in short-term culture.

### DETAILED DESCRIPTION

This description of preferred embodiments is intended to be read in connection with the accompanying drawings, which are to be considered part of the entire written description of this invention. The drawing figures are not necessarily to scale and certain features of the invention may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship. When only a single machine is illustrated, the term "machine" shall also be taken to include any collection of machines that individually or jointly execute a set (or multiple sets) of instructions to perform any one or more of the methodologies discussed herein. In the claims, means-plus-function clauses, if used, are intended to cover the structures described, suggested, or rendered obvious by the written description or drawings for performing the recited function, including not only structural equivalents but also equivalent structures. The references to the methods of treatment in this description are to be interpreted as references to compositions of the present invention for use in those methods.

The present invention is based, in part, on the our discovery that Bcl2-associated athanogene 3 (BAG3) regulates contractility and Ca²⁺ homeostasis in ventricular myocytes. More specifically, we have found that BAG3 is localized in the sarcolemma of adult myocytes, as opposed to the cytoplasm of neonatal myocytes, and that BAG3 regulates contractility and Ca²⁺ homeostasis in adult ventricular myocytes by virtue of its selective association with the β- adrenergic receptor. We have found that in the adult cardiac myocyte, BAG3 facilitates the ability of β-adrenergic signaling to augment cardiac contraction through alterations in Ca²⁺ homeostasis. More specifically, we have found that BAG3 modulates myocyte contraction and action potential duration by regulating the interactions between β-adrenergic receptor signaling and [Ca2+]ᵢ homeostasis. Unlike neonatal cardiomyocytes in which BAG3 distributed diffusely in the cytoplasm, in adult myocytes BAG3 co-localized with Na⁺-K⁺-ATPase in the sarcolemma and t-tubules. BAG3 also co-immunoprecipitated with β 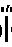 -adrenergic receptor, L-type Ca²⁺ channels and phospholemman in extracts from adult myocytes. BAG3 downregulation by shRNA (shBAG3) had no effect on myocyte contraction and [Ca2+]ᵢ dynamics at baseline but resulted in reduced myocyte contraction amplitudes, lower systolic [Ca2+]ᵢ and [Ca2+]ᵢ transient amplitudes after stimulation with isoproterenol. L-type Ca²⁺ current (I_{Ca}) and sarcoplasmic reticulum (SR) Ca²⁺ content but not Na⁺/ Ca²⁺ exchange current (iNaCa) or SR Ca²⁺ uptake were reduced in isoproterenol -treated shBAG3 myocytes. Forskolin or dibutyrl cAMP restored ICa amplitude in shBAG3 myocytes to that observed in WT myocytes. Resting membrane potential and action potential amplitude were unaffected but APD50 and APD90 were prolonged in shBAG3 myocytes. Expression of alc-subunit of L-type Ca²⁺ channel, SR Ca²⁺-ATPase, Na⁺/ Ca²⁺ exchanger, α1- and α2-subunits of Na⁺-K⁺-ATPase, and phosphorylated ryanodine receptor was unchanged in shBAG3 myocytes. In addition, BAG3 overexpression resulted in enhanced myocyte contractility in the presence of isoproterenol. Finally, we found that hypoxia/reoxygenation injury resulted in the translocation of BAG3 from sarcolemma to the cytoplasm. Our finding that BAG3 was expressed at the sarcolemma and t-tubules and modulated myocyte contraction in the presence of isoproterenol provides a paradigm for contractile dysfunction and insensitivity to β-adrenergic stimulation that characterizes hearts with HFrEF and low levels of BAG3.

Accordingly, the invention features compositions comprising a nucleic acid encoding a BAG3 polypeptide or biologically active variant thereof according to claim 1. Also featured are methods of administering the compositions to enhance cardiac performance, for example, to increase cardiac contractility, in a patient suffering from HFrEF or at risk for HFrEF. In some embodiments, the patient expresses low levels of BAG3. In some embodiments, the patient harbors a BAG3 mutation. The therapeutic methods described herein can be carried out in connection with other heart failure treatments, for example, drug therapies or medical devices.

### Compositions

Bcl2-associated athanogene 3 (BAG3) is a stress-activated 575 amino acid protein that is abundantly expressed in the heart, skeletal muscles and many cancers. BAG3 is constitutively expressed in the heart and skeletal muscle and to a lesser extent in organs containing extensive smooth muscle including the uterus, bladder and aorta. A member of the 6-member BAG family of proteins, BAG3 regulates protein quality control (PQC) by serving as a co-chaperone of partner proteins including the constitutively and non-constitutively expressed heat shock proteins (Hsc/Hsp) and has anti-apoptotic effects mediated through binding to Bcl2. BAG3 plays a role in the stability of the sarcomere through regulation of filamin clearance and production and by binding to the actin capping protein beta 1(CapZb1), a sarcomere protein that binds to the barbed end of actin to prevent its disassociation into actin monomers.

BAG3 expression is markedly enhanced in the presence of stress such as heat-shock, hypoxia and chemicals and in many neoplastic cells. A co-chaperone of the constitutive and non-constitutively expressed heat shock proteins (Hsc/Hsp70), BAG3 is critical for maintaining PQC by degrading misfolded and aggregated proteins via macroautophagy through the formation of a ternary complex composed of BAG3, Hsc/Hsp70, and the C-terminus of the Hsc70-interacting protein (CHIP) and through chaperon-assisted macro-autophagy (CASA)(2). BAG3 also protects cells from apoptotic death by binding to Bcl2 and promotes structural stability of filamentous actin (F-actin) by enhancing association between Hsp70 and CapZβ1. BAG3 stimulates filamin transcription by using its WW domain to engage inhibitors of the transcriptional activators YAP and TAZ - changes that allow cancer cells to metastasize.

Mice with homozygous deletion of the BAG3 gene had postnatal deterioration with death by 4 weeks of age due to non-inflammatory myofibrillar degeneration. Functional mutations in BAG3 were found in childhood-onset muscular dystrophy with involvement of skeletal, respiratory and cardiac muscles, in families with dilated cardiomyopathy but without neuropathy or peripheral muscle weakness, and in sporadic cases of idiopathic dilated cardiomyopathy. BAG3 protein levels in hearts from patients with end-stage heart failure (HF) but without known BAG3 mutations were significantly less than those measured in non-failing control hearts.

BAG3, also known as MFM6; Bcl-2-Binding Protein Bis;CAIR-1; Docking Protein CAIR-1; BAG Family Molecular Chaperone Regulator 3; BAG-3; BCL2-Binding Athanogene 3; or BIS, is a cytoprotective polypeptide that competes with Hip-1 for binding to HSP 70. The NCBI reference amino acid sequence for BAG3 can be found at Genbank under accession number NP_004272.2; Public GI: 14043024. We refer to the amino acid sequence of Genbank accession number NP_004272.2; Public GI: 14043024 as SEQ ID NO: 1. The NCBI reference nucleic acid sequence for BAG3 can be found at Genbank under accession number NM_004281.3 GI:62530382. We refer to the nucleic acid sequence of Genbank accession number NM_004281.3 GI:62530382 as SEQ ID NO: 2. Other BAG3 amino acid sequences include, for example, without limitation, 095817.3 GI:12643665 (SEQ ID NO: 3); EAW49383.1 GI:119569768 (SEQ ID NO: 4); EAW49382.1 Gl:119569767(SEQ ID NO: 5); and CAE55998.1 GI:38502170 (SEQ ID NO: 6). The BAG3 polypeptide of the invention can be a variant of a polypeptide described herein, provided it retains functionality.

### Polypeptides

The compositions of the invention can include a BAG3 polypeptide encoded by any of the nucleic acid sequences described above. The terms "peptide," "polypeptide," and "protein" are used interchangeably herein, although typically they refer to peptide sequences of varying sizes. We may refer to the amino acid-based compositions of the invention as "polypeptides" to convey that they are linear polymers of amino acid residues, and to help distinguish them from full-length proteins. The invention encompasses polypeptides that constitute or include biologically active variants of BAG3 as defined in the claims. will be understood that the polypeptides can therefore include only a biologically active variant of BAG3 but may include additional residues as well. Biologically active variants will retain sufficient activity to cleave target DNA.

The bonds between the amino acid residues can be conventional peptide bonds or another covalent bond (such as an ester or ether bond), and the polypeptides can be modified by amidation, phosphorylation or glycosylation. A modification can affect the polypeptide backbone and/or one or more side chains. Chemical modifications can be naturally occurring modifications made in vivo following translation of an mRNA encoding the polypeptide (e.g., glycosylation in a bacterial host) or synthetic modifications made in vitro. A biologically active variant of BAG3 can include one or more structural modifications resulting from any combination of naturally occurring (i.e., made naturally in vivo) and with synthetic modifications (i.e., naturally occurring or non-naturally occurring modifications made in vitro). Examples of modifications include, but are not limited to, amidation (e.g., replacement of the free carboxyl group at the C-terminus by an amino group); biotinylation (e.g., acylation of lysine or other reactive amino acid residues with a biotin molecule); glycosylation (e.g., addition of a glycosyl group to either asparagines, hydroxylysine, serine or threonine residues to generate a glycoprotein or glycopeptide); acetylation (e.g., the addition of an acetyl group, typically at the N-terminus of a polypeptide); alkylation (e.g., the addition of an alkyl group); isoprenylation (e.g., the addition of an isoprenoid group); lipoylation (e.g. attachment of a lipoate moiety); and phosphorylation (e.g., addition of a phosphate group to serine, tyrosine, threonine or histidine).

One or more of the amino acid residues in a biologically active variant may be a non-naturally occurring amino acid residue. Naturally occurring amino acid residues include those naturally encoded by the genetic code as well as non-standard amino acids (e.g., amino acids having the D-configuration instead of the L-configuration). The present peptides can also include amino acid residues that are modified versions of standard residues (e.g. pyrrolysine can be used in place of lysine and selenocysteine can be used in place of cysteine). Non-naturally occurring amino acid residues are those that have not been found in nature, but that conform to the basic formula of an amino acid and can be incorporated into a peptide. These include D-alloisoleucine(2R,3S)-2-amino-3-methylpentanoic acid and L-cyclopentyl glycine (S)-2-amino-2-cyclopentyl acetic acid. For other examples, one can consult textbooks or the worldwide web (a site is currently maintained by the California Institute of Technology and displays structures of non-natural amino acids that have been successfully incorporated into functional proteins).

Alternatively, or in addition, one or more of the amino acid residues in a biologically active variant can be a naturally occurring residue that differs from the naturally occurring residue found in the corresponding position in a wildtype sequence. In other words, biologically active variants can include one or more amino acid substitutions. We may refer to a substitution, addition, or deletion of amino acid residues as a mutation of the wildtype sequence. As noted, the substitution can replace a naturally occurring amino acid residue with a non-naturally occurring residue or just a different naturally occurring residue. Further the substitution can constitute a conservative or non-conservative substitution. Conservative amino acid substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The polypeptides that are biologically active variants of BAG3 can be characterized in terms of the extent to which their sequence is similar to or identical to the corresponding wild-type polypeptide. A biologically active variant of BAG3 has an amino acid sequence with at least 90%, 95%, 97%, 98%, or 99% sequence identity to BAG3 or to a homolog or ortholog thereof.

A biologically active variant of a BAG3 polypeptide will retain sufficient biological activity to be useful in the present methods. The biologically active variants will retain sufficient activity to function in targeted DNA cleavage. The biological activity can be assessed in ways known to one of ordinary skill in the art and includes, without limitation, in vitro cleavage assays or functional assays.

Polypeptides can be generated by a variety of methods including, for example, recombinant techniques or chemical synthesis. Once generated, polypeptides can be isolated and purified to any desired extent by means well known in the art. For example, one can use lyophilization following, for example, reversed phase (preferably) or normal phase HPLC, or size exclusion or partition chromatography on polysaccharide gel media such as Sephadex G-25. The composition of the final polypeptide may be confirmed by amino acid analysis after degradation of the peptide by standard means, by amino acid sequencing, or by FAB-MS techniques. Salts, including acid salts, esters, amides, and N-acyl derivatives of an amino group of a polypeptide may be prepared using methods known in the art, and such peptides are useful in the context of the present invention.

### Nucleic acids

We may use the terms "nucleic acid" and "polynucleotide" interchangeably to refer to both RNA and DNA, including cDNA, genomic DNA, synthetic DNA, and DNA (or RNA) containing nucleic acid analogs, any of which may encode a polypeptide of the invention and all of which are encompassed by the invention. Polynucleotides can have essentially any three-dimensional structure. A nucleic acid can be double-stranded or single-stranded (i.e., a sense strand or an antisense strand). Non-limiting examples of polynucleotides include genes, gene fragments, exons, introns, messenger RNA (mRNA) and portions thereof, transfer RNA, ribosomal RNA, siRNA, micro-RNA, ribozymes, cDNA, recombinant polynucleotides, branched polynucleotides, plasmids, vectors, isolated DNA of any sequence, isolated RNA of any sequence, nucleic acid probes, and primers, as well as nucleic acid analogs. In the context of the present invention, nucleic acids can encode a biologically active variant of a naturally occurring BAG3.

An "isolated" nucleic acid can be, for example, a naturally-occurring DNA molecule or a fragment thereof, provided that at least one of the nucleic acid sequences normally found immediately flanking that DNA molecule in a naturally-occurring genome is removed or absent. Thus, an isolated nucleic acid includes, without limitation, a DNA molecule that exists as a separate molecule, independent of other sequences (e.g., a chemically synthesized nucleic acid, or a cDNA or genomic DNA fragment produced by the polymerase chain reaction (PCR) or restriction endonuclease treatment). An isolated nucleic acid also refers to a DNA molecule that is incorporated into a vector, an autonomously replicating plasmid, a virus, or into the genomic DNA of a prokaryote or eukaryote. In addition, an isolated nucleic acid can include an engineered nucleic acid such as a DNA molecule that is part of a hybrid or fusion nucleic acid. A nucleic acid existing among many (e.g., dozens, or hundreds to millions) of other nucleic acids within, for example, cDNA libraries or genomic libraries, or gel slices containing a genomic DNA restriction digest, is not an isolated nucleic acid.

Isolated nucleic acid molecules can be produced by standard techniques. For example, polymerase chain reaction (PCR) techniques can be used to obtain an isolated nucleic acid containing a nucleotide sequence described herein, including nucleotide sequences encoding a polypeptide described herein. PCR can be used to amplify specific sequences from DNA as well as RNA, including sequences from total genomic DNA or total cellular RNA. Various PCR methods are described in, for example, PCR Primer: A Laboratory Manual, Dieffenbach and Dveksler, eds., Cold Spring Harbor Laboratory Press, 1995. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid.

Isolated nucleic acids also can be chemically synthesized, either as a single nucleic acid molecule (e.g., using automated DNA synthesis in the 3' to 5' direction using phosphoramidite technology) or as a series of oligonucleotides. For example, one or more pairs of long oligonucleotides (e.g., >50-100 nucleotides) can be synthesized that contain the desired sequence, with each pair containing a short segment of complementarity (e.g., about 15 nucleotides) such that a duplex is formed when the oligonucleotide pair is annealed. DNA polymerase is used to extend the oligonucleotides, resulting in a single, double-stranded nucleic acid molecule per oligonucleotide pair, which then can be ligated into a vector. Isolated nucleic acids of the invention also can be obtained by mutagenesis of, e.g., a naturally occurring portion of a BAG3 -encoding DNA (in accordance with, for example, the formula above).

Two nucleic acids or the polypeptides they encode may be described as having a certain degree of identity to one another. For example, a BAG3 protein and a biologically active variant thereof may be described as exhibiting a certain degree of identity. Alignments may be assembled by locating short BAG3 sequences in the Protein Information Research (PIR) site (http://pir.georgetown.edu), followed by analysis with the "short nearly identical sequences" Basic Local Alignment Search Tool (BLAST) algorithm on the NCBI website (http://www.ncbi.nlm.nih.gov/blast).

As used herein, the term "percent sequence identity" refers to the degree of identity between any given query sequence and a subject sequence. For example, a naturally occurring BAG3 can be the query sequence and a fragment of a BAG3 protein can be the subject sequence. Similarly, a fragment of a B AG3 protein can be the query sequence and a biologically active variant thereof can be the subject sequence.

To determine sequence identity, a query nucleic acid or amino acid sequence can be aligned to one or more subject nucleic acid or amino acid sequences, respectively, using the computer program ClustalW (version 1.83, default parameters), which allows alignments of nucleic acid or protein sequences to be carried out across their entire length (global alignment).

ClustalW calculates the best match between a query and one or more subject sequences and aligns them so that identities, similarities and differences can be determined. Gaps of one or more residues can be inserted into a query sequence, a subject sequence, or both, to maximize sequence alignments. For fast pair wise alignment of nucleic acid sequences, the following default parameters are used: word size: 2; window size: 4; scoring method: percentage; number of top diagonals: 4; and gap penalty: 5. For multiple alignments of nucleic acid sequences, the following parameters are used: gap opening penalty: 10.0; gap extension penalty: 5.0; and weight transitions: yes. For fast pair wise alignment of protein sequences, the following parameters are used: word size: 1; window size: 5; scoring method: percentage; number of top diagonals: 5; gap penalty: 3. For multiple alignment of protein sequences, the following parameters are used: weight matrix: blosum; gap opening penalty: 10.0; gap extension penalty: 0.05; hydrophilic gaps: on; hydrophilic residues: Gly, Pro, Ser, Asn, Asp, Gin, Glu, Arg, and Lys; residue-specific gap penalties: on. The output is a sequence alignment that reflects the relationship between sequences. ClustalW can be run, for example, at the Baylor College of Medicine Search Launcher site (searchlauncher.bcm.tmc.edu/multi-align/multi-align.html) and at the European Bioinformatics Institute site on the World Wide Web (ebi.ac.uk/clustalw).

To determine a percent identity between a query sequence and a subject sequence, ClustalW divides the number of identities in the best alignment by the number of residues compared (gap positions are excluded), and multiplies the result by 100. The output is the percent identity of the subject sequence with respect to the query sequence. It is noted that the percent identity value can be rounded to the nearest tenth. For example, 78.11, 78.12, 78.13, and 78.14 are rounded down to 78.1, while 78.15, 78.16, 78.17, 78.18, and 78.19 are rounded up to 78.2.

The nucleic acids and polypeptides described herein may be referred to as "exogenous". The term "exogenous" indicates that the nucleic acid or polypeptide is part of, or encoded by, a recombinant nucleic acid construct, or is not in its natural environment. For example, an exogenous nucleic acid can be a sequence from one species introduced into another species, i.e., a heterologous nucleic acid. Typically, such an exogenous nucleic acid is introduced into the other species via a recombinant nucleic acid construct. An exogenous nucleic acid can also be a sequence that is native to an organism and that has been reintroduced into cells of that organism. An exogenous nucleic acid that includes a native sequence can often be distinguished from the naturally occurring sequence by the presence of non-natural sequences linked to the exogenous nucleic acid, e.g., non-native regulatory sequences flanking a native sequence in a recombinant nucleic acid construct. In addition, stably transformed exogenous nucleic acids typically are integrated at positions other than the position where the native sequence is found.

Recombinant constructs are also provided herein and can be used to transform cells in order to express BAG3. A recombinant nucleic acid construct comprises a nucleic acid encoding a BAG3 sequence operably linked to a regulatory region suitable for expressing the BAG3 in the particular cell. It will be appreciated that a number of nucleic acids can encode a polypeptide having a particular amino acid sequence. The degeneracy of the genetic code is well known in the art. For many amino acids, there is more than one nucleotide triplet that serves as the codon for the amino acid. For example, codons in the coding sequence for BAG3 can be modified such that optimal expression in a particular organism is obtained, using appropriate codon bias tables for that organism.

Vectors containing nucleic acids such as those described herein also are provided. A "vector" is a replicon, such as a plasmid, phage, or cosmid, into which another DNA segment may be inserted so as to bring about the replication of the inserted segment. Generally, a vector is capable of replication when associated with the proper control elements. Suitable vector backbones include, for example, those routinely used in the art such as plasmids, viruses, artificial chromosomes, BACs, YACs, or PACs. The term "vector" includes cloning and expression vectors, as well as viral vectors and integrating vectors. An "expression vector" is a vector that includes a regulatory region. A wide variety of host/expression vector combinations may be used to express the nucleic acid sequences described herein. Suitable expression vectors include, without limitation, plasmids and viral vectors derived from, for example, bacteriophage, baculoviruses, and retroviruses.

Useful vectors include, for example, viral vectors (such as adenoviruses ("Ad"), adeno-associated viruses (AAV), lentiviruses, and vesicular stomatitis virus (VSV) and retroviruses). Replication-defective recombinant adenoviral vectors, can also be used. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003).

A "recombinant viral vector" refers to a viral vector comprising one or more heterologous gene products or sequences. Since many viral vectors exhibit size-constraints associated with packaging, the heterologous gene products or sequences are typically introduced by replacing one or more portions of the viral genome. Such viruses may become replication-defective, requiring the deleted function(s) to be provided in trans during viral replication and encapsidation (by using, e.g., a helper virus or a packaging cell line carrying gene products necessary for replication and/or encapsidation). Modified viral vectors in which a polynucleotide to be delivered is carried on the outside of the viral particle have also been described.

Viral vectors can include a strong eukaryotic promoter operably linked to the polynucleotide e.g., a cytomegalovirus (CMV) promoter. The recombinant viral vector can include one or more of the polynucleotides therein, preferably about one polynucleotide. In some embodiments, the viral vector used in the invention methods has a pfu (plague forming units) of from about 10⁸ to about 5x 10¹⁰ pfu. In embodiments in which the polynucleotide is to be administered with a non-viral vector, use of between from about 0.1 nanograms to about 4000 micrograms will often be useful e.g., about 1 nanogram to about 100 micrograms.

Additional vectors include retroviral vectors such as Moloney murine leukemia viruses and HIV-based viruses. One HIV-based viral vector comprises at least two vectors wherein the gag and pol genes are from an HIV genome and the env gene is from another virus. DNA viral vectors include pox vectors such as orthopox or avipox vectors, herpesvirus vectors such as a herpes simplex I virus (HSV) vector.

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors may be an indication for some invention embodiments. The adenovirus vector results in a shorter term expression (e.g., less than about a month) than adeno-associated virus, in some embodiments, may exhibit much longer expression. The particular vector chosen will depend upon the target cell and the condition being treated. The selection of appropriate promoters can readily be accomplished. An example of a suitable promoter is the 763-base-pair cytomegalovirus (CMV) promoter.

Other suitable promoters which may be used for gene expression include, but are not limited to, the Rous sarcoma virus (RSV), the SV40 early promoter region, the herpes thymidine kinase promoter, the regulatory sequences of the metallothionein (MMT) gene, prokaryotic expression vectors such as the β-lactamase promoter, the tac promoter, promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells, insulin gene control region which is active in pancreatic beta cells, immunoglobulin gene control region which is active in lymphoid cells, mouse mammary tumor virus control region which is active in testicular, breast, lymphoid and mast cells, albumin gene control region which is active in liver, alpha-fetoprotein gene control region which is active in liver, alpha 1-antitrypsin gene control region which is active in the liver, beta-globin gene control region which is active in myeloid cells, myelin basic protein gene control region which is active in oligodendrocyte cells in the brain, myosin light chain-2 gene control region which is active in skeletal muscle, and gonadotropic releasing hormone gene control region which is active in the hypothalamus. Certain proteins can expressed using their native promoter. Other elements that can enhance expression can also be included such as an enhancer or a system that results in high levels of expression such as a tat gene and tar element. This cassette can then be inserted into a vector, e.g., a plasmid vector such as, pUC19, pUC118, pBR322, or other known plasmid vectors, that includes, for example, an E. coli origin of replication. The plasmid vector may also include a selectable marker such as the β-lactamase gene for ampicillin resistance, provided that the marker polypeptide does not adversely affect the metabolism of the organism being treated. The cassette can also be bound to a nucleic acid binding moiety in a synthetic delivery system.

In some embodiments, the compositions of the invention include nucleic acids encoding a CRISPR- associated endonuclease, e.g., Cas9, and a guide RNA that is complementary to a target sequence in BAG3. The crRNA and tracrRNA can be expressed separately or engineered into an artificial fusion small guide RNA (sgRNA) via a synthetic stem loop (AGAAAU) to mimic the natural crRNA/tracrRNA duplex. Such sgRNA, like shRNA, can be synthesized or in vitro transcribed for direct RNA transfection or expressed from an expression vector.

In some embodiments, delivery systems can include a peripheral intravenous injection with a vector that selectively transduces only cardiomyocytes, for example, AAV serotypes that have strong cardiac tropism. Other systems involving percutaneous and surgical techniques include, for example, antegrade intra-coronary infusion either with or without coronary artery occlusion; closed-loop recirculation, wherein the vector is infused into a coronary artery removed from the circulation from the coronary sinus oxygenated extracorporeally and redeliver down the coronary artery; retrograde infusion through coronary sinus; direct myocardial injection; peripheral intravenous infusion; and pericardial injection.

In some embodiments, the polynucleotides of the invention may also be used with a microdelivery vehicle such as cationic liposomes, other lipid-containing complexes, and other macromolecular complexes capable of mediating delivery of a polynucleotide to a host cell.

Another delivery method is to use single stranded DNA producing vectors which can produce the expressed products intracellularly. See for example, Chen et al, BioTechniques, 34: 167-171 (2003), which is incorporated herein, by reference, in its entirety.

The vectors provided herein also can include, for example, origins of replication, scaffold attachment regions (SARs), and/or markers. A marker gene can confer a selectable phenotype on a host cell. For example, a marker can confer biocide resistance, such as resistance to an antibiotic (e.g., kanamycin, G418, bleomycin, or hygromycin). As noted above, an expression vector can include a tag sequence designed to facilitate manipulation or detection (e.g., purification or localization) of the expressed polypeptide. Tag sequences, such as green fluorescent protein (GFP), glutathione S-transferase (GST), polyhistidine, c-myc, hemagglutinin, or Flag^{™} tag (Kodak, New Haven, CT) sequences typically are expressed as a fusion with the encoded polypeptide. Such tags can be inserted anywhere within the polypeptide, including at either the carboxyl or amino terminus.

Additional expression vectors also can include, for example, segments of chromosomal, non-chromosomal and synthetic DNA sequences. Suitable vectors include derivatives of SV40 and known bacterial plasmids, e.g., E. coli plasmids col E1, pCR1, pBR322, pMal-C2, pET, pGEX, pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2µ plasmid or derivatives thereof, vectors useful in eukaryotic cells, such as vectors useful in insect or mammalian cells; vectors derived from combinations of plasmids and phage DNAs, such as plasmids that have been modified to employ phage DNA or other expression control sequences.

The vector can also include a regulatory region. The term "regulatory region" refers to nucleotide sequences that influence transcription or translation initiation and rate, and stability and/or mobility of a transcription or translation product. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, nuclear localization signals, and introns.

As used herein, the term "operably linked" refers to positioning of a regulatory region and a sequence to be transcribed in a nucleic acid so as to influence transcription or translation of such a sequence. For example, to bring a coding sequence under the control of a promoter, the translation initiation site of the translational reading frame of the polypeptide is typically positioned between one and about fifty nucleotides downstream of the promoter. A promoter can, however, be positioned as much as about 5,000 nucleotides upstream of the translation initiation site or about 2,000 nucleotides upstream of the transcription start site. A promoter typically comprises at least a core (basal) promoter. A promoter also may include at least one control element, such as an enhancer sequence, an upstream element or an upstream activation region (UAR). The choice of promoters to be included depends upon several factors, including, but not limited to, efficiency, selectability, inducibility, desired expression level, and cell- or tissue-preferential expression. It is a routine matter for one of skill in the art to modulate the expression of a coding sequence by appropriately selecting and positioning promoters and other regulatory regions relative to the coding sequence.

Regardless of whether compositions are administered as nucleic acids or polypeptides, they are formulated in such a way as to promote uptake by the mammalian cell. Useful vector systems and formulations are described above. In some embodiments the vector can deliver the compositions to a specific cell type. The invention is not so limited however, and other methods of DNA delivery such as chemical transfection, using, for example calcium phosphate, DEAE dextran, liposomes, lipoplexes, surfactants, and perfluoro chemical liquids are also contemplated, as are physical delivery methods, such as electroporation, micro injection, ballistic particles, and "gene gun" systems.

### Methods of Treatment

The compositions disclosed herein are generally and variously useful for treatment of a subject having heart failure. We may refer to a subject, patient, or individual interchangeably. Heart failure generally occurs when the heart is unable to maintain sufficient blood flow to meet the body's needs. Heart failure is also described as congestive heart failure because a common symptom is swelling or water retention. Heart failure can be classified as chronic heart failure- in which the patient remains stable over time with treatment- or acute heart failure-in which the patient experiences a sudden onset of symptoms that may include shortness of breath, weakness or fatigue.

Heart failure is the final common stage of many different diseases of the heart. Heart failure can be divided into two types: heart failure due to left ventricular dysfunction and heart failure with normal ejection fraction. While we believe we understand certain events that occur upon administration of compositions comprising a nucleic acid encoding a BAG3 polypeptide or fragment thereof, the compositions of the present invention are not limited to those that work by affecting any particular cellular mechanism. Our working hypothesis is that administration of a BAG3 polypeptide or fragment thereof to failing heart tissue may enhance cardiac performance by stimulating cardiac contractility, while at the same time limiting apoptosis and enhancing autophagy.

Symptoms of heart failure can include shortness of breath, fatigue and weakness, edema of legs and ankles and feet, rapid or irregular heartbeat, reduced ability to exercise, persistent cough or wheezing, increased urination at night, ascites, sudden weight gain, nausea, confusion or difficulty concentrating, elevated heart rate, sudden severe shortness of breath, and chest pain.

The methods are useful for the treatment of diseases or disorders that can result in heart failure, e.g., HFrEF, for example, nonischemic cardiomyopathy, nonischemic dilated cardiomyopathy, idiopathic dilated cardiomyopathy or familial dilated cardiomyopathy. In some embodiments, the methods are broadly useful for the treatment of cardiomyopathy. Cardiomyopathy encompasses a range of myocardial disorders in which the heart muscle is structurally abnormal and functions abnormally. Exemplary cardiomyopathies include primary/intrinsic cardiomyopathy, for example dilated cardiomyopathy, and secondary cardiomyopathies for example cardiomyopathies due to metabolic disorders; inflammation resulting from viral or parasitic infections; endocrine disorders such as diabetes; toxicity resulting from chemotherapy or alcoholism; neuromuscular disorders such as muscular dystrophy; nutritional diseases; genetic disorders, for example disorders in which sarcomere genes have been mutated or deleted, including but not limited to mutations in the BAG3 gene.

In some embodiments, the patient's BAG3 status can be determined prior to treatment. The BAG3 gene can be sequenced to determine whether the patient harbors a BAG3 mutation.

Risk factors for heart failure can vary. Exemplary clinical risk factors include age, gender hypertension, left ventricular hypertrophy, myocardial infarction, valvular heart disease, and diabetes. Other exemplary clinical risk factors include smoking, dyslipidemia, chronic kidney disease, albuminuria, increased heart rate, dietary risk factors, sedentary lifestyle, socioeconomic status, and psychological stress. Risk factors include immune mediated factors, such as peripartum cardiomyopathy, hypersensitivity; infectious disease mediated factors, for example viral parasitic or bacterial infections; toxic risk precipitants, for example chemotherapy alcohol or cocaine use. Genetic risk factors include family history of congenital heart disease. Biomarkers may also be useful in identification of risk factors.

A subject is effectively treated whenever a clinically beneficial result ensues. This may mean, for example, a complete resolution of the symptoms of a disease, a decrease in the severity of the symptoms of the disease, or a slowing of the disease's progression. These methods can further include the steps of a) identifying a subject (e.g., a patient and, more specifically, a human patient) who has or who is at risk for heart failure; and b) providing to the subject a therapeutically effective amount of a composition comprising a nucleic acid encoding a BAG3 polypeptide or fragment thereof. A subject can be identified using standard clinical tests, for example, blood tests, chest x-rays, and electrocardiogram (ECG), an echocardiogram, a stress test, a CT scan, MRI, or cardiac catheterization. An amount of such a composition provided to the subject that results in a complete resolution of the symptoms of the infection, a decrease in the severity of the symptoms of the infection, or a slowing of the infection's progression is considered a therapeutically effective amount. The present methods may also include a monitoring step to help optimize dosing and scheduling as well as predict outcome.

The methods disclosed herein can be applied to a wide range of species, e.g., humans, non-human primates (e.g., monkeys), horses or other livestock, dogs, cats, ferrets or other mammals kept as pets, rats, mice, or other laboratory animals.

The methods of the invention can be expressed in terms of the preparation of a medicament. Accordingly, the invention encompasses the use of the agents and compositions described herein in the preparation of a medicament. The compounds described herein are useful in therapeutic compositions and regimens or for the manufacture of a medicament for use in treatment of diseases or conditions as described herein.

Any composition described herein can be administered to any part of the host's body for subsequent delivery to a target cell. A composition can be delivered to, without limitation, the heart, the brain, the cerebrospinal fluid, joints, nasal mucosa, blood, lungs, intestines, muscle tissues, skin, or the peritoneal cavity of a mammal. In terms of routes of delivery, a composition can be administered by intravenous, intracranial, intraperitoneal, intramuscular, subcutaneous, intramuscular, intrarectal, intravaginal, intrathecal, intratracheal, intradermal, or transdermal injection, by oral or nasal administration, or by gradual perfusion over time. In a further example, an aerosol preparation of a composition can be given to a host by inhalation.

The dosage required will depend on the route of administration, the nature of the formulation, the nature of the patient's illness, the patient's size, weight, surface area, age, and sex, other drugs being administered, and the judgment of the attending clinicians. Wide variations in the needed dosage are to be expected in view of the variety of cellular targets and the differing efficiencies of various routes of administration. Variations in these dosage levels can be adjusted using standard empirical routines for optimization, as is well understood in the art. Administrations can be single or multiple (e.g., 2- or 3-, 4-, 6-, 8-, 10-, 20-, 50-, 100-, 150-, or more fold). Encapsulation of the compounds in a suitable delivery vehicle (e.g., polymeric microparticles or implantable devices) may increase the efficiency of delivery.

The duration of treatment with any composition provided herein can be any length of time from as short as one day to as long as the life span of the host (e.g., many years). For example, a compound can be administered once a week (for, for example, 4 weeks to many months or years); once a month (for, for example, three to twelve months or for many years); or once a year for a period of 5 years, ten years, or longer. It is also noted that the frequency of treatment can be variable. For example, the present compounds can be administered once (or twice, three times, etc.) daily, weekly, monthly, or yearly.

An effective amount of any composition provided herein can be administered to an individual in need of treatment. The term "effective" as used herein refers to any amount that induces a desired response while not inducing significant toxicity in the patient. Such an amount can be determined by assessing a patient's response after administration of a known amount of a particular composition. In addition, the level of toxicity, if any, can be determined by assessing a patient's clinical symptoms before and after administering a known amount of a particular composition. It is noted that the effective amount of a particular composition administered to a patient can be adjusted according to a desired outcome as well as the patient's response and level of toxicity. Significant toxicity can vary for each particular patient and depends on multiple factors including, without limitation, the patient's disease state, age, and tolerance to side effects.

Any method known to those in the art can be used to determine if a particular response is induced. Clinical methods that can assess the degree of a particular disease state can be used to determine if a response is induced. The particular methods used to evaluate a response will depend upon the nature of the patient's disorder, the patient's age, and sex, other drugs being administered, and the judgment of the attending clinician.

The compositions may also be administered with another therapeutic agent. Exemplary agents include angiotensin-converting enzyme (ACE) inhibitors, angiotensin II receptor blockers, beta blockers, aldosterone antagonists, hydralazine, isosorbide dinitrate diuretics, digoxin, and blood thinning medications such as aspirin or warfarin. The compositions can be administered with a beta-adrenergic agonist in patients with an acute exacerbation of heart failure.

The compositions may also be administered in conjunction with the use of a medical device. Exemplary medical devices include biventricular pacemakers, implantable cardiac devices such as implantable cardiac defibrillators, pacemakers for use in cardiac resynchronization therapy, and left ventricular assist devices.

Concurrent administration of two or more therapeutic agents does not require that the agents be administered at the same time or by the same route, as long as there is an overlap in the time period during which the agents are exerting their therapeutic effect. Simultaneous or sequential administration is contemplated, as is administration on different days or weeks. The therapeutic agents may be administered under a metronomic regimen, e.g., continuous low-doses of a therapeutic agent.

Dosage, toxicity and therapeutic efficacy of such compositions can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀.

The data obtained from the cell culture assays and animal studies can be used in formulating a range of dosage for use in humans. The dosage of such compositions lies preferably within a range of circulating concentrations that include the ED₅₀ with little or no toxicity. The dosage may vary within this range depending upon the dosage form employed and the route of administration utilized. For any composition used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Articles of manufacture

The compositions described herein can be packaged in suitable containers labeled, for example, for use as a therapy to treat a subject having a suffering from or at risk for heart failure. The containers can include a composition comprising a nucleic acid sequence encoding a BAG3 polypeptide or fragment thereof or a vector encoding that nucleic acid, and one or more of a suitable stabilizer, carrier molecule, flavoring, and/or the like, as appropriate for the intended use. Accordingly, packaged products (e.g., sterile containers containing one or more of the compositions described herein and packaged for storage, shipment, or sale at concentrated or ready-to-use concentrations) and kits, including at least one composition of the invention, e.g., a nucleic acid sequence encoding a BAG3 polypeptide or fragment thereof or a vector encoding that nucleic acid . A product can include a container (e.g., a vial, jar, bottle, bag, or the like) containing one or more compositions of the invention. In addition, an article of manufacture further may include, for example, packaging materials, instructions for use, syringes, delivery devices, buffers or other control reagents for treating or monitoring the condition for which prophylaxis or treatment is required.

In some embodiments, the kits can include one or more additional therapeutic agents. The additional agents can be packaged together in the same container as a nucleic acid sequence encoding a BAG3 polypeptide or fragment thereof or a vector encoding that nucleic acid or they can be packaged separately. The nucleic acid sequence encoding a BAG3 polypeptide or fragment thereof or a vector encoding that nucleic acid .and the additional agent may be combined just before use or administered separately.

The product may also include a legend (e.g., a printed label or insert or other medium describing the product's use (e.g., an audio- or videotape)). The legend can be associated with the container (e.g., affixed to the container) and can describe the manner in which the compositions therein should be administered (e.g., the frequency and route of administration), indications therefor, and other uses. The compositions can be ready for administration (e.g., present in dose-appropriate units), and may include one or more additional pharmaceutically acceptable adjuvants, carriers or other diluents and/or an additional therapeutic agent. Alternatively, the compositions can be provided in a concentrated form with a diluent and instructions for dilution.

### EXAMPLES

### Example 1: Materials and Methods

Construction of Adv-BAG3-shRNA. BAG3shRNA-Ad construct was made using the BD Adeno-X Expression Systems 2PT3674-1 (Pr36024) and BD knockout RNAi Systems PT3739 (PR42756)(BD Biosciences-Clontech, Palo Alto, CA) as previously described. A dsDNA oligonucleotide against a specific BAG3 mRNA (5'-AAG GUU CAG ACC AUC UUG GAA-3') was inserted in a RNAi -ready pSIREN-DNR vector designed to express a small hairpin RNA (shRNA) driven by the human Pol III- dependent U6 promoter. After ligation, this vector was used to transfer the shRNA expression cassette to the Adenoviral Acceptor Vector pLP-Adeno-X-PRLS viral DNA (BD Adeno-X Expression Systems 2) containing ΔE1/ΔE3 Ad5 genome by Cre-loxP mediated recombination. An AdNull empty adenoviral vector was used as control. Adenoviruses were propagated in a HEK-293 cell line, purified and titered (plaque-forming unit; pfu) according to standard techniques.

Isolation, adenoviral infection and culture of adult murine cardiac myocytes. Cardiac myocytes were isolated from the septum and LV free wall of C57BL/6 mice (10-12 wks old) according to the protocol of Zhou et al., and plated on laminin-coated glass coverslips. Two hours after isolation, myocytes were infected with replication-deficient adenovirus (Adv) expressing green fluorescent protein (GFP)(6.6 x 10⁷ pfu/ml), Adv-GFP-myc-tagged human BAG3 (1.1 x 10⁷ pfu/ml) or Adv-GFP-shRNA BAG3 (2.0 x 10⁸ pfu/ml) in 1 ml of fetal bovine serum (FBS)-free Eagle minimal essential medium (MEM) containing 0.2 % bovine serum albumin, creatine (5 mM), carnitine (2 mM), taurine (5 mM), NaHC03 (4.2 mM), penicillin (30 mg/L), gentamicin (4 mg/L), insulin-transferrin-selenium supplement and 2,3-butanedione monoxime (BDM, 10 mM) for 3h. An additional ml of MEM (with same supplements) was then added, and myocytes were cultured for 48h before measurements. Media was changed daily. We have previously demonstrated that under our culture conditions, adult mouse myocytes cultured for up to 48h maintained rod-shape morphology, t-tubule organization and normal contractile function (40). Before measurements of contraction, [Ca2+]ᵢ transients, INaCa, ICa, AP and SR Ca²⁺ content, culture medium containing BDM was aspirated, cells were bathed with MEM without BDM and returned to the incubator (37°C) for 30 min.

Coverslips containing cultured myocytes were mounted in Dvorak-Stotler chamber, and bathed in fresh media before measurements. For the sake of brevity, myocytes infected with Adv-GFP, Adv-GFP-BAG3- myc and Adv-GFP-shRNA-BAG3 are referred to as GFP, BAG3 and shBAG3 myocytes, respectively.

Immunolocalization of BAG 3 in adult LV myocytes. Freshly isolated WT mouse LV myocytes were plated on laminin-coated coverslips, allowed to adhere for 3h, and washed 3x with phosphate buffered saline containing 2 mM EGTA (PBS-E). Myocytes were fixed for 30 min in 4% paraformaldehyde in PBS-E. After 2 rinses with PBS-E, myocytes were permeabilized for 2 min with 0.05% Triton X-100. Myocytes were rinsed 2x with PBS-E, and once with BLOTTO (5% nonfat dry milk, 0.1 M NaCl, and 50 mM Tris.HCl; pH 7.4). Primary antibodies against BAG3 (1:50; Bethyl Labs, Montgomery, TX) diluted in BLOTTO were added to the cells, incubated at room temperature in the dark for 60 min, and rinsed 3x with BLOTTO. Secondary antibodies (Alexafluor 594-labeled goat anti-rabbit IgG; 1:50; Invitrogen, Eugene, OR) diluted in BLOTTO were added to the cells, incubated in the dark for 30 min, and followed by 3 PBS-E rinses. Coverslips were mounted to slides with Prolong Gold Anti-fade mounting solution (Invitrogen). Confocal images (63 × oil objective; 510 Meta; Carl Zeiss, Inc.) were acquired at 594 nm excitation and 617 nm emission for BAG3. For comparison, endogenous BAG3 expression pattern in neonatal rat ventricular myocytes (NRVM) was examined.

In another series of experiments, freshly isolated mouse LV myocytes were incubated in Krebs- Henseleit Bicarbonate (KHB) buffer (37°C) containing pyruvate (5 mM) as the sole substrate under either normoxic (21 % O₂) or hypoxic (1% O₂) conditions for 30 min. KHB buffer was replaced with MEM containing supplements and myocytes were returned to culture for 2 or 48h before endogenous BAG3 localization was examined.

In a 3^{rd} series of experiments, isolated adult myocytes were infected with Adv-GFP-BAG3-myc and Adv-β1 adrenergic receptor (β1APv) tagged with HA and cultured for 48h. BAG3 was detected with Alexafluor 555-labeled anti-myc antibodies (1:250; Millipore; ex. 561 nm, em. >575 nm), and Na⁺-K⁺- ATPase was detected with Alexafluor 488-labeled anti-α1 subunit (1:250; Millipore; ex. 488 nm, em. 510 nm).

Knockdown of BAG 3 by Adv-shRNA BAG3 injection into LV. After cleansing the skin with betadine solution, the left chest of anesthetized (2% inhaled isoflurane) mouse was opened, the heart exteriorized, and 35 µl (total volume) of Adv-GFP (3.3 x 10 pfu) or Adv-shRNA BAG3 (7.5 x10 pfu) was directly injected to anterior and posterior LV wall and the apex. The hear1 was returned to the chest cavity and the wound sutured. The entire surgical procedure took <45 seconds. Typically >95% of animals survived the procedure. Survivors were allowed to recover for 7 to 10 days before hearts were excised and myocytes were isolated from areas of LV that fluoresced green (indicating successful adenovirus-mediated gene transfer.

Measurement of [Ca ²⁺ ]i and contraction in cardiac myocytes. Fura-2 loaded (0.67 µM fura-2 AM, 15 min) myocytes adherent to laminin-coated coverslips were incubated in HEPES-buffered (20 mM, pH 7.4) medium 199 (1.8 mM [Ca²⁺]ₒ) and field stimulated to contract (2 Hz; 37°C). Myocytes were exposed to excitation light (360 and 380 nm) only during data acquisition. Epifluorescence (510 nm) was measured in steady-state twitches both before and after addition of isoproterenol (Iso; 1 µM). Daily calibration of fura-2 signals and [Ca2+]ᵢ transient analyses were performed as previously described. For contraction measurements, images of myocytes (not loaded with fura-2) were captured by a charge coupled device video camera and myocyte motion was analyzed offline with edge detection algorithm as previously described.

INaCa, ICa, AP and SR Ca²⁺ content measurements. Whole cell patch-clamp recordings were performed at 30°C as described previously. Pipette diameter was 4 - 6 µm and pipette resistance was 0.8 to 1.4 MΩ when filled with standard internal solution. For INaCa measurements, pipette solution contained (in mM): Cs⁺-glutamate 100, Na⁺-HEPES 7.25, MgCl2 1, HEPES 12.75, Na₂ATP 2.5, EGTA 10, and CaCl₂ 6; pH 7.2. Free Ca²⁺ in the pipette solution was 205 nM. Myocytes were bathed in an external solution containing (in mM): NaCl 130, CsCl 5, MgSO₄ 1.2, NaH₂PO₄ 1.2, CaCl₂ 5, HEPES 10, Na⁺-HEPES 10, and glucose 10; pH 7.4 Verapamil (1 µM) was added to block L-type Ca²⁺ currents. The myocyte was held at the calculated equilibrium potential for INaCa (ENaCa) of -73 mV for at least 5 min before current was elicited with a descending-ascending voltage ramp (from +100 to -120 and back to +100 mV, 500 mV/s). INaCa was defined as the difference current in the absence and presence of NiCl2 (1 mM). Our conditions for measuring INaCa were carefully chosen to minimize contamination by Na⁺-K⁺- ATPase activity (K⁺-free) and ion fluxes through the Na /Ca²⁺ exchanger before the onset of voltage ramp (by holding the cell at the calculated ENaCa), thereby allowing [NA⁺]i and [Ca2+]ᵢ to equilibrate with those present in the pipette solution.

For ICa measurements, pipette solution contained (in mM): CsCl 110, TEA. CI 20, HEPES 10, MgATP 5, and EGTA 10; pH 7.2. Extracellular bathing solution contained (in mM): N-methyl-D-glucamine 137, CsCl 5.4, CaCl₂ 2, MgSO₄ 1.3, HEPES 20, 4-aminopyridine 4, and glucose 15; pH 7.4. Our solutions were designed to be Na⁺- and K⁺-free. Holding potential was at -90 mV. To ensure steady-state SR Ca²⁺ loading, 6 conditioning pulses (from -70 to 0 mV, 100 ms, 2 Hz) were delivered to the myocyte before the arrival of each test pulse (from -90 to +50 mV, 10 mV increments, 60 ms). Leak- subtracted inward currents were used in analysis for ICa amplitudes and inactivation kinetics. Inward currents obtained under these conditions were blocked by 1 µM verapamil (data not shown). In some experiments, ICa was measured both in the absence and presence of 1 µM isoproterenol. In other experiments, ICa was measured before and after addition of forskolin (10 µM) or dibutyrl cAMP (5 mM).

For AP measurements, myocytes were paced at 1 Hz. Pipette solution consisted of (in mM): KCl125, MgCl₂ 4, CaCl₂ 0.06, HEPES 10, K⁺-EGTA 5, Na₂ATP 3, and Na₂-creatine phosphate 5, (pH 7.2). External solution consisted of (in mM): NaCl 132, KCI 5.4, CaCl₂ 1.8, MgCl2 1.8, NaH₂PO₄ 0.6, HEPES 7.5, Na⁺-HEPES 7.5, and glucose 5, pH 7.4. APs were recorded using the current clamp configuration at 1.5x threshold stimulus, 4-ms duration, and 30°C.

SR Ca²⁺ content was estimated by integrating forward INaCa induced by caffeine exposure as described previously. The pipette solution consisted of (in mM): Cs⁺-glutamate 100, MgCl2 1, HEPES 30 and MgATP 2.5, pH 7.2. The external solution contained (in mM): NaCl 130, CsCl 5, MgSO₄ 1.2, NaH₂PO₄ 1.2, CaCl₂ 1.8, HEPES 20, glucose 10, pH 7.4; 30°C. Holding potential was -90 mV. At 200ms after the 12^{th} conditioning pulse (from -90 to 0 mV, 300ms, 1 Hz), with membrane potential (Eₘ) held at -90 mV, caffeine (5 mM, 2.4s) was applied. The resulting inward current was digitized at 0.5 kHz and collected for 5 s. To convert INaCa time integral (coulombs) to moles, charge was divided by Faraday's constant of 96,487 coulomb s/equivalent, based on 3 Na⁺ being exchanged for each Ca²⁺. In some experiments, SR Ca²⁺ content was measured in myocytes stimulated with isoproterenol (1 µM) before the prepulses. I_{Ca}, I_{NaCa} and SR ca²⁺ contents were normalized to membrane capacitance (Cm) before comparison between GFP and shBAG3 myocytes.

Immunoblotting. Mouse LV homogenates were prepared as previously described (39). Gradient (4-12%) gels were used in all Western blots. For detection of BAG3, α1 - and α2- subunits of Na⁺-K⁺-ATPase, α1c- subunit of L-type Ca²⁺ channel (Caᵥ1.2), sarco(endo)plasmic reticulum Ca²⁺-ATPase (SERCA2), calsequestrin and cardiac ryanodine receptor phosphorylated at serine²⁸⁰⁸ (pRyR2), reducing conditions (5% β-mercaptoethanol) was used. For detection of Na⁺/ Ca²⁺ exchanger (NCX1), non-reducing conditions (10 mM N-ethylmaleimide) was used. Rabbit antihuman BAG3 polyclonal antibody was obtained from Proteintech Group, Inc. (Chicago, IL), while antibodies for others were sourced and used as described previously. Blots were washed and incubated with appropriate secondary antibody conjugated to horse radish peroxidase. Enhanced chemiluminescence (ECL, Amersham) was used for the detection of signals.

Co-immunoprecipitation. Isolated adult myocytes were infected with Adv-GFP-BAG3-myc and Adv-β1AR-HA or Adv-GFP (control) and cultured for 48h. Myocytes were scraped into lysis buffer (in mM): 50 Tris (pH 8.0), 150 NaCl, 1 Na⁺ orthovanadate, 1 PMSF, 100 NaF, 1 EDTA and 1 EGTA with 0.5% NP-40, 10 µg/ml leupeptin and 10 µg/ml aprotinin, sonicated and centrifuged at 10,000rpm and 4°C for 10 min. 40ul (50% slurry) monoclonal anti-HA-agarose beads were added to 600µl of supernatant (400 µg protein) and incubated overnight on a rotating platform at 4°C. Beads were pelleted and washed 4x with 1ml ice-cold PBS. 52 ul of 2x loading buffer were added to the beads, and incubated at room temperature for 30 min with slow vortex every 5 min. The bead suspension was centrifuged at 5000g for 1 min and 40 µl of the supernatant were collected. Supernatant was heated at 70°C for 5 min before immunoblotting (7.5% SDS-PAGE, reducing conditions with 5% β-mercaptoethanol). Blots were incubated with primary anti-HA. antibody (1:1,000) followed by antimouse secondary antibody (1:15,000; Li-Cor). β1AR-HA co-immunoprecipitates were detected by anti-BAG3 antibody (1:1,000), anti-α1c-Cav1.2 antibody (1:200), anti-a1-Na⁺-K⁺-ATPase (1:1,000); anti-PLM antibody (C2, 1:1,000), anti-CapZβ1 (1:500), and anti-Hsp70 (1:500). Protein band signals were detected by enhanced chemiluminescence or Li-Cor.

Statistics. All results are expressed as means ± SE. For analysis of systolic and diastolic [Ca²⁺]i, [Ca2+]ᵢ transient amplitudes, myocyte contraction amplitudes, and SR Ca²⁺ contents as a function of group and Iso; INaCa and ICa as a function of group and voltage, two-way ANOVA was used. For analysis of action potential parameters and protein abundance, one-way ANOVA was used. A commercially available software package (JMP version 7; SAS Institute, Cary, NC) was used. In all analyses, p<0.05 was taken to be statistically significant.

### Example 2: BAG3 is localized in sarcolemma and t-tubules in adult LV cardiac myocytes but in the cytoplasm in neonatal rat ventricular myocytes (NRVM)

Immunol ocalization experiments using anti-BAG3 antibodies in freshly isolated adult mouse LV myocytes demonstrated that endogenous BAG3 was expressed in sarcolemma and t-tubules (Fig. 1), suggesting that BAG3 may interact with sarcolemmal signal transduction proteins and/or ion transporters. By contrast, in NRVMs, BAG3 was expressed diffusely in the cytoplasm rather than localized in the plasma membrane (Fig. 1). In addition, in cultured adult myocytes expressing myc-tagged human BAG3, anti-myc antibodies showed that BAG3 localized with Na⁺-K⁺-ATPase (Fig. 2), thereby providing additional support that BAG3 localized in the plasma membrane of adult myocytes.

### Example 3: BAG3 downregulation depresses myocyte contractility and reduces [Ca2+]ᵢ transient amplitudes

To simulate reduced BAG3 protein levels in HF (1, 11), we initially attempted to downregulate BAG3 by infecting isolated adult myocytes with Adv-shRNA BAG3 followed by culture for 48h. This did not result in any decrease in BAG3 protein levels (Fig. 3A), suggesting more than 48h was required for shRNA BAG3 to knockdown BAG3 in adult myocytes.

Since adult mouse LV myocytes do not maintain their contractility and rod-shape in long-term culture, we attempted to downregulate BAG3 by injecting Adv-shRNA BAG3 in intact myocardium. Hearts injected with Adv-GFP served as controls. Seven to ten days after virus injection, BAG3 levels significantly decreased in shBAG3 compared to GFP myocytes (Fig. 3A). BAG3 knockdown did not alter expression of α1- and α2-subunits of Na⁺-K⁺-ATPase, Na⁺/Ca²⁺ exchanger, a1c-subunit of Caᵥ1.2, pRyR2 and SERCA2 (Fig. 3B; Table 1). In addition, there were no significant differences (p<0.6) in cell sizes as reflected by similar whole cell capacitance (Cm, a measure of cell surface area) between GFP (163.1 ± 5.4 pF; n=20) and shBAG3 (167.7 ± 5.7 pF; n=22) myocytes.

With BAG3 knockdown, single myocyte contraction amplitudes were similar at baseline but were significantly lower in shBAG3 compared to control GFP myocytes when stimulated with isoproterenol (Fig. 3C; Table 2; group x iso interaction effect, p<0.01). Likewise, systolic [Ca2+]ᵢ and [Ca2+]ᵢ transient amplitudes were similar at baseline but significantly reduced in shBAG3 compared to GFP myocytes after isoproterenol (Fig. 3C; Table 2; group x iso interaction effect, p<0.0003 for systolic [Ca2+]ᵢ and p<0.03 for [Ca2+]ᵢ transient amplitude).

There were no differences in diastolic [Ca2+]ᵢ and t1/2 of [Ca2+]ᵢ transient decline (Table 2), suggesting similar SR Ca ²⁺ uptake rates between GFP and shBAG3 myocytes.

### Example 4: Effects of BAG3 downregulation on INaCa, ICa and SR Ca2+ contents

The major determinants of [Ca2+]ᵢ transient amplitude are the triggers for SR Ca²⁺ release (ICa and to a much lesser extent, reverse INaCa) and SR Ca²⁺ content. Since INaCa was not affected by isoproterenol in adult rodent cardiac myocytes, we measured INaCa at baseline and found no differences between GFP and shBAG3 myocytes (Fig. 4A).

Under basal conditions, maximal ICa amplitude and voltage at which ICa peaked were similar in control GFP and shBAG3 myocytes (Fig. 4B). The inactivation time constant of ICa measured at - 10 mV (xinact) was similar between GFP (12.2 ± 0.8 ms; n=12) and shBAG3 myocytes (13.3 ± 0.63 ms; n=21)(p<0.3). By contrast, in the presence of isoproterenol, maximal ICa amplitude was significantly (p<0.02; group x Iso interaction effect) lower in shBAG3 myocytes (Fig. 4B; Table 3) but xinact was similar between GFP (8.0 ± 0.7 ms; n=5) and shBAG3 (10.0 ± 1.5 ms; n=8)(p<0.25) myocytes. To determine if the shBAG3- mediated decrease in responsiveness to Iso was due to a generalized defect in cAMP signaling, we tested whether direct activation of adenylyl cyclase (forskolin) or protein kinase A (dibutyryl cAMP) also produced diminished ICa responses. Addition of either forskolin (10 µM) or dibutyryl cAMP (5 mM) produced maximal ICa amplitudes in shBAG3 myocytes that were not different than those observed in GFP myocytes stimulated with Iso (Table 3). SR Ca²⁺ contents were not different between GFP and shBAG3 myocytes at baseline but were lower in shBAG3 myocytes in the presence of isoproterenol (Fig. 4C; Table 2). In addition, t1/2 of INaCa decline after caffeine-induced SR Ca²⁺ release (an alternative measure of NCX1 activity) was similar between GFP and shBAG3 myocytes (Fig. 4C; Table 2), in agreement with there being no difference in INaCa measured in GFP and shBAG3 myocytes (Fig. 4A).

Example 5: BAG3 associates with β 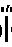 -adrenergic receptor, L-type Ca ²⁺ channel and phospholemman in adult LV myocytes.

Since BAG3 downregulation resulted in diminished ICa amplitudes in adult myocytes stimulated with isoproterenol (Fig. 4B), but signaling pathways distal to β1AR were intact in shBAG3 myocytes (Table 3), we evaluated if there was physical interaction between β1AR and BAG3. In myocytes expressing both β1AR-HA and BAG3-myc, the exogenous BAG3 (detected with anti-myc antibody) was correctly targeted to the plasma membrane (Fig. 2). Immunoprecipitation with anti-HA antibody brought down not only β1AR-HA (as expected) but also BAG3, Caᵥ1.2 and phospholemman (PLM) but not Na⁺-K⁺-ATPase (Fig. 5A). PLM, which belongs to the FXYD family of ion transport regulators, is expressed in the heart and regulates Na⁺-K⁺-ATPase, Na⁺/Ca²⁺ exchanger and L-type Ca²⁺ channel in adult cardiac myocytes. By contrast, immunoprecipitating βI-AR with anti- HA antibody did not bring down CapZb1 or Hsp70 (Fig. 5B) in adult LV cardiac myocytes.

### Example 6: BAG3 downregulation prolongs action potential duration

Resting membrane potential and action potential amplitude were similar between GFP and shBAG3 myocytes, both in the absence and presence of isoproterenol (Fig. 6). BAG3 downregulation resulted in prolongation of APD50 (p<0.03) and APD90 (p<0.05) (Fig. 6), both in the absence and presence of isoproterenol.

### Example 7: Effects of BAG3 overexpression on myocyte and cardiac contractility

Since BAG3 depletion reduced cardiac myocyte responsiveness to isoproterenol, we next sought to determine whether overexpression of BAG3 could, conversely, enhance β1AR responsiveness. Two days after Adv-BAG3 infection, BAG3 levels were 67.0 ± 5.2 arbitrary units (a.u.) in BAG3 myocytes compared to 10.3 ±1.0 a.u. in control GFP myocytes (p<0.0004 ; Fig. 7A). There were no differences in expression of al-subunit of Na⁺-K⁺-ATPase, alc-subunit of Caᵥ1.2, and SERCA2 between GFP and BAG3 myocytes (Fig. 7A). At baseline, maximal contraction amplitudes and contraction dynamics were not different between GFP and BAG3 myocytes (Fig. 7B; Table 4). However, after stimulation with isoproterenol, maximal contraction amplitudes were significantly (p<0.04) higher and maximal re-lengthening velocities were significantly (p<0.01) faster in BAG3 myocytes (Fig. 7B; Table 4).

### Example 8: Effects of hypoxia/reoxygenation on BAG3 localization in adult myocytes.

Since the dominant physiological role of BAG3 in normal adult cardiac myocytes appears to be regulation of excitation- contraction coupling and β 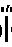 adrenergic responsiveness in the sarcolemma, we imposed hypoxia/reoxygenation injury to evaluate if BAG3 translocates to cytoplasm and peri-nuclear region. In well-oxygenated myocytes cultured for 2 and 48h, BAG3 remained localized in the sarcolemma and t- tubules with little-to-no signal in the cytoplasm (Figs. 8A & B). By contrast, adult myocytes that were exposed to 30 min. of hypoxia demonstrated redistribution of BAG3 in the cytoplasm not at 2 hours (Fig.8C) but after 48h of culture (Fig. 8D).

The identification of a new role for BAG3 in the heart is based on four fundamental observations. First, by contrast with NRVMs, confocal imaging demonstrated BAG3 in the sarcolemma and t-tubules, with little-to-no signal in the sarcoplasm in adult mouse cardiac myocytes. This observation was supported by the finding that BAG3 associated with β1 adrenergic receptor, L-type Ca²⁺ channels and phospholemman but not Na⁺-K⁺-ATPase and is consistent with previous reports that there is a separate pool of phospholemman that does not regulate or associate with the Na⁺ pump. In sharp contrast to studies in NRVMs, BAG3 associated with sarcolemmal β1ARs but did not associate with Hsp70 or CapZβ1 in adult cardiac myocytes. Our finding that BAG3 co-localized with the Z-disc in NRVMs is consistent with two earlier studies in neonatal cardiomyocytes in which BAG3 co-localized with the Z- disc under basal conditions. BAG3 knockdown had no effect on normal neonatal cardiomyocytes but destabilized myocyte structure and caused disruption of myofibril structures when cardiomycoytes were stretched. Our finding that BAG3 is located in the plasma membrane in adult cardiac myocytes under resting conditions is surprising. Previous studies have only used neonatal cardiomyocytes. However, when adult cardiac myocytes were stressed with hypoxia and re-oxygenation, BAG3 translocated to the cytoplasm and peri-nuclear regions, co-localizing with the contractile proteins and the autophagy machinery. The marked differences between BAG3 localization in NRVMs and adult cardiac myocytes is consistent with the observation in cardiomyoblasts that the functional role of BAG3 changes with different developmental stages while the shift in localization of BAG3 in the presence of cell stress is consistent with studies in non-myocytes.

The second major finding is that adenoviral delivery of shRNA-BAG3 was efficacious in knocking down BAG3 levels by -54% in adult mouse hearts after 7-10 days of infection - a decrease comparable to that seen in human hearts with end-stage failure and in animal models of HF. The observation that BAG3 levels were unchanged after 2 days of Adv-shRNA-BAG3 infection suggests that the turnover of BAG3 in adult mouse myocytes is relatively slow. By comparison, anti-sense oligonucleotides targeted to the start codon (nucleotides -11 to +9) of guinea pig cardiac Na⁺/Ca²⁺ exchanger mRNA resulted in 40% decrease in NCX1 expression after only 2 days of exposure.

The final substantive observation is that BAG3 downregulation to -50% of that found in normal WT myocytes resulted in reduced maximum contraction amplitude, shortening and relengthening velocities in response to βAR stimulation, an effect that was not associated with myocyte hypertrophy (similar Cm) or altered expression levels of protein involved in Ca²⁺ dynamics. By contrast, BAG3 overexpression with Adv-BAG3 enhanced contractility of myocytes when stimulated with isoproterenol while not altering expression levels of proteins associated with cardiac excitation-contraction coupling. Taken together, these results strongly suggest that BAG3 plays a critical role in modulating cardiac response to catecholamines.

The major determinants of systolic [Ca2+]ᵢ and [Ca2+]ᵢ transient amplitudes are the trigger for SR Ca²⁺ release (ICa and reverse INaCa), the gain of ryanodine receptor, and SR Ca²⁺ content. To understand the mechanism responsible for alterations in myocyte contractility, we investigated changes in Ca²⁺ homeostasis in single myocytes. BAG3 downregulation resulted in decreased ICa and SR Ca²⁺ content but not INaCa in myocytes treated with isoproterenol. However, ICa was not reduced in shBAG3 myocytes exposed to either forskolin or dbcAMP, suggesting defects in β-adrenergic receptor signaling occurred proximal to adenylyl cyclase. This conclusion is given circumstantial support in that BAG3, β1AR, L-type Ca²⁺ channel physically associated with each other and likely formed a macromolecular signaling complex in adult cardiac myocytes. Decreased SR Ca²⁺ content in shBAG3 myocytes were not due to reduced SR Ca²⁺ uptake since SERCA2 expression was not affected by BAG3 downregulation and SR Ca²⁺ uptake activity, as reflected by t1/2 of [Ca2+]ᵢ transient decline, was similar between GFP and shBAG3 myocytes. The lower SR Ca ²⁺ content in shBAG3 myocytes exposed to isoproterenol was most likely due to decreased SR Ca²⁺ loading by ICa since both INaCa and pRyR2 expression (SR Ca²⁺ leak) were not affected by BAG3 downregulation. Another piece of evidence that Na⁺/Ca²⁺ exchange activity was unaltered by BAG3 downregulation is the observation that t1/2 of INaCa decline after caffeine-induced Ca₂₊ release was similar between control GFP and shBAG3 myocytes.

An unexpected finding was that BAG3 downregulation resulted in prolongation of the AP in myocytes, regardless of whether they had been treated with isoproterenol. Since AP morphology and duration are largely dependent on voltage-dependent ion currents, prolongation of the AP is another line of evidence that BAG3 modulated sarcolemmal ion channel activity. In addition, since cell sizes were similar between GFP and shBAG3 myocytes, altered AP morphology is a primary effect of BAG3 downregulation rather than a secondary effect associated with myocyte hypertrophy. In shBAG3 myocytes, reduction in transient outward current (Iₜₒ) could cause APD50 prolongation as observed in post-infarct and failing rodent myocytes, while reduction in depolarization-activated K⁺ currents could result in APD₉₀ lengthening. Since AP prolongation is associated with increased risks of arrhythmias, BAG3 reduction may account for increased risks of sudden death in patients with familial dilated cardiomyopathy and patients with end-stage heart failure.

## Claims

1. A composition for therapeutic use in enhancing cardiac performance by increasing or enhancing cardiac contractility in a patient suffering from heart failure, the composition comprising:
a pharmaceutical formulation comprising a nucleic acid encoding a BAG3 polypeptide or biologically active variant thereof with an amino acid sequence at least 90% identical to BAG3.

2. The composition for therapeutic use of claim 1, wherein the patient has non-ischemic cardiomyopathy.

3. The composition for therapeutic use of claim 2, wherein the patient has a mutated BAG3 gene.

4. The composition for therapeutic use of claim 1, wherein the patient has cardiomyopathy.

5. The composition for therapeutic use of claim 2, wherein the non-ischemic cardiomyopathy is idiopathic dilated cardiomyopathy or familial dilated cardiomyopathy.

6. The composition for therapeutic use of claim 1, wherein the heart failure is acute heart failure.

7. The composition for therapeutic use of claim 1, wherein the nucleic acid encoding the BAG3 polypeptide is operably linked to an expression vector.

8. The composition for therapeutic use of claim 7, wherein the expression vector comprises an adeno-associated virus vector (AAV).

9. The composition for therapeutic use of claim 7, wherein the expression vector is a cardiotropic vector.

10. The composition for therapeutic use of claim 7, wherein the expression vector comprises a tissue specific regulatory region.

11. The composition for therapeutic use of claim 1, wherein the enhanced cardiac performance comprises an increase in myocyte contraction amplitude, an increase in myocyte shortening velocity, an increase in myocyte relengthening velocity or a combination thereof.

12. The composition for therapeutic use of claim 1, wherein the composition comprises a pharmaceutically acceptable carrier.

13. The composition for therapeutic use of claim 1, wherein the patient is human.

14. The composition for therapeutic use of claim 1, wherein the patient has ischemic cardiomyopathy.

15. The composition for therapeutic use of claim 1, wherein the nucleic acid encodes a biologically active variant of the BAG3 polypeptide with an amino acid sequence at least 97% identical to BAG3.

16. The composition for therapeutic use of claim 1, wherein the nucleic acid encodes a biologically active variant of the BAG3 polypeptide with an amino acid sequence at least 98% identical to BAG3.

17. The composition for therapeutic use of claim 1, wherein the nucleic acid encodes a biologically active variant of the BAG3 polypeptide with an amino acid sequence at least 99% identical to BAG3.

## Patentansprüche

1. Zusammensetzung zur therapeutischen Anwendung zur Verbesserung der Leistungsfähigkeit des Herzens durch Steigerung oder Verstärkung der Herzkontraktilität bei einem Patienten, der an Herzinsuffizienz leidet, wobei die Zusammensetzung Folgendes umfasst:
eine pharmazeutische Formulierung, die eine Nukleinsäure umfasst, die für ein BAG3-Polypeptid oder eine biologisch aktive Variante davon mit einer zu mindestens 90 % mit BAG3 identischen Aminosäuresequenz kodiert.

2. Zusammensetzung zur therapeutischen Verwendung nach Anspruch 1, wobei der Patient an einer nicht-ischämischen Kardiomyopathie leidet.

3. Zusammensetzung zur therapeutischen Verwendung nach Anspruch 2, wobei der Patient eine Mutation im BAG3-Gen aufweist.

4. Zusammensetzung zur therapeutischen Verwendung nach Anspruch 1, wobei der Patient an einer Kardiomyopathie leidet.

5. Zusammensetzung zur therapeutischen Verwendung nach Anspruch 2, wobei es sich bei der nicht-ischämischen Kardiomyopathie um eine idiopathische dilatative Kardiomyopathie oder eine familiäre dilatative Kardiomyopathie handelt.

6. Zusammensetzung zur therapeutischen Verwendung nach Anspruch 1, wobei es sich bei der Herzinsuffizienz um eine akute Herzinsuffizienz handelt.

7. Zusammensetzung zur therapeutischen Verwendung nach Anspruch 1, wobei die für das BAG3-Polypeptid kodierende Nukleinsäure funktionsfähig an einen Expressionsvektor gebunden ist.

8. Zusammensetzung zur therapeutischen Verwendung nach Anspruch 7, wobei der Expressionsvektor einen Adeno-assoziierten Virusvektor (AAV) umfasst.

9. Zusammensetzung zur therapeutischen Verwendung nach Anspruch 7, wobei der Expressionsvektor ein kardiotroper Vektor ist.

10. Zusammensetzung zur therapeutischen Verwendung nach Anspruch 7, wobei der Expressionsvektor eine gewebsspezifische regulatorische Region umfasst.

11. Zusammensetzung zur therapeutischen Verwendung nach Anspruch 1, wobei die verbesserte Leistungsfähigkeit des Herzens eine Erhöhung der Kontraktionsamplitude der Myozyten, eine Erhöhung der Kontraktionsgeschwindigkeit der Myozyten, eine Erhöhung der Relaxtionsgeschwindigkeit der Myozyten oder eine Kombination davon umfasst.

12. Zusammensetzung zur therapeutischen Verwendung nach Anspruch 1, wobei die Zusammensetzung einen pharmazeutisch verträglichen Träger umfasst.

13. Zusammensetzung zur therapeutischen Verwendung nach Anspruch 1, wobei es sich bei dem Patienten um einen Menschen handelt.

14. Zusammensetzung zur therapeutischen Verwendung nach Anspruch 1, wobei der Patient an einer ischämischen Kardiomyopathie leidet.

15. Zusammensetzung zur therapeutischen Verwendung nach Anspruch 1, wobei die Nukleinsäure für eine biologisch aktive Variante des BAG3-Polypeptids mit einer zu mindestens 97 % mit BAG3 identischen Aminosäuresequenz kodiert.

16. Zusammensetzung zur therapeutischen Verwendung nach Anspruch 1, wobei die Nukleinsäure eine biologisch aktive Variante des BAG3-Polypeptids mit einer zu mindestens 98 % mit BAG3 identischen Aminosäuresequenz kodiert.

17. Zusammensetzung zur therapeutischen Verwendung nach Anspruch 1, wobei die Nukleinsäure eine biologisch aktive Variante des BAG3-Polypeptids mit einer zu mindestens 99 % mit BAG3 identischen Aminosäuresequenz kodiert.

## Revendications

1. Composition à usage thérapeutique destinée à améliorer les performances cardiaques en augmentant ou en renforçant la contractilité cardiaque chez un patient souffrant d'insuffisance cardiaque, la composition comprenant :
une formulation pharmaceutique incluant un acide nucléique codant un polypeptide BAG3 ou un variant biologiquement actif de celui-ci dont la séquence d'acides aminés présente au moins 90 % d'identité avec BAG3.

2. Composition à usage thérapeutique selon la revendication 1, dans laquelle le patient présente une cardiomyopathie non ischémique.

3. Composition à usage thérapeutique selon la revendication 2, dans laquelle le patient présente une mutation du gène BAG3.

4. Composition à usage thérapeutique selon la revendication 1, dans laquelle le patient présente une cardiomyopathie.

5. Composition à usage thérapeutique selon la revendication 2, dans laquelle la cardiomyopathie non ischémique est une cardiomyopathie dilatée idiopathique ou une cardiomyopathie dilatée familiale.

6. Composition à usage thérapeutique selon la revendication 1, dans laquelle l'insuffisance cardiaque est une insuffisance cardiaque aiguë.

7. Composition à usage thérapeutique selon la revendication 1, dans laquelle l'acide nucléique codant le polypeptide BAG3 est lié de manière fonctionnelle à un vecteur d'expression.

8. Composition à usage thérapeutique selon la revendication 7, dans laquelle le vecteur d'expression comprend un vecteur viral adéno-associé (AAV).

9. Composition à usage thérapeutique selon la revendication 7, dans laquelle le vecteur d'expression est un vecteur viral cardiotrope.

10. Composition à usage thérapeutique selon la revendication 7, dans laquelle le vecteur d'expression comprend une région régulatrice spécifique du tissu.

11. Composition à usage thérapeutique selon la revendication 1, dans laquelle l'amélioration des performances cardiaques comprend une augmentation de l'amplitude de contraction des myocytes, une augmentation de la vitesse de raccourcissement des myocytes, une augmentation de la vitesse de réallongement des myocytes, ou une combinaison de celles-ci.

12. Composition à usage thérapeutique selon la revendication 1, dans laquelle la composition comprend un véhicule pharmaceutiquement acceptable.

13. Composition à usage thérapeutique selon la revendication 1, dans laquelle le patient est humain.

14. Composition à usage thérapeutique selon la revendication 1, dans laquelle le patient présente une cardiomyopathie ischémique.

15. Composition à usage thérapeutique selon la revendication 1, dans laquelle l'acide nucléique code un variant biologiquement actif du polypeptide BAG3 dont la séquence d'acides aminés présente au moins 97 % d'identité avec BAG3.

16. Composition à usage thérapeutique selon la revendication 1, dans laquelle l'acide nucléique code un variant biologiquement actif du polypeptide BAG3 dont la séquence d'acides aminés présente au moins 98 % d'identité avec BAG3.

17. Composition à usage thérapeutique selon la revendication 1, dans laquelle l'acide nucléique code un variant biologiquement actif du polypeptide BAG3 dont la séquence d'acides aminés présente au moins 99 % d'identité avec BAG3.
